(19) Europäisches Patentamt
European Patent Office
Office européen des brevets

(11) Numéro de publication: **0 034 546**
**B1**

(12) # FASCICULE DE BREVET EUROPEEN

(45) Date de publication du fascicule du brevet: **16.05.84**

(21) Numéro de dépôt: **81400272.1**

(22) Date de dépôt: **20.02.81**

(51) Int. Cl.³: **C 07 D 498/06,** A 61 K 31/535 // C07D209/94 ,(C07D498/06, 265/00, 209/00)

(54) **Nouveaux dérivés du cycloheptindole et leurs sels d'addition avec les acides, leur préparation, leur application comme médicaments et les compositions les renfermant.**

(30) Priorité: **26.02.80 FR 8004198**

(43) Date de publication de la demande: **26.08.81 Bulletin 81/34**

(45) Mention de la délivrance du brevet: **16.05.84 Bulletin 84/20**

(84) Etats contractants désignés: **AT BE CH DE FR GB IT LI LU NL SE**

(56) Documents cités: **Néant**

(73) Titulaire: **ROUSSEL-UCLAF, 35, boulevard des Invalides, F-75007 Paris (FR)**

(72) Inventeur: **Nedelec, Lucien, 45, boulevard de l'Ouest, F-93340 Le Raincy (FR)**
Inventeur: **Frechet, Daniel, 45, rue Lecourbe, F-75015 Paris (FR)**
Inventeur: **Dumont, Claude, 33, rue du Maréchal Vaillant, F-94130 Nogent Sur Marne (FR)**

(74) Mandataire: **Douetteau, Pierre et al, c/o ROUSSEL-UCLAF 102, route de Noisy Boite postale 9, F-93230 Romainville (FR)**

EP 0 034 546 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Nouveaux dérivée du cycloheptindole et leurs sels d'addition avec les acides, leur préparation, leur application comme médicaments et les compositions les renfermant

La présente invention concerne de nouveaux dérivés du cycloheptindole ainsi que leurs sels, leur procédé de préparation, l'application à titre de médicaments de ces nouveaux dérivés et les compositions les renfermant.

L'invention a pour objets les nouveaux dérivés du cycloheptindole, ainsi que leurs sels d'addition avec les acides minéraux ou organiques, caractérisés en ce qu'ils répondent à la formule générale I:

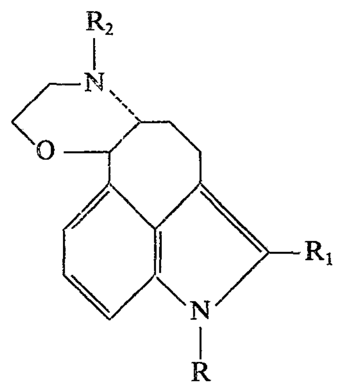

(I)

dans laquelle R représente un atome d'hydrogène, un radical alcoyle renfermant de 1 à 5 atomes de carbone, ou un radical aralcoyle renfermant de 7 à 12 atomes de carbone éventuellement substitué par des radicaux chloro, bromo, méthyl, éthyl, méthoxy, trifluorométhyl ou méthylthio, $R_1$ représente un atome d'hydrogène, de chlore ou de brome, et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio.

Dans la formule générale I et dans ce qui suit, le terme radical alcoyle renfermant de 1 à 5 atomes de carbone désigne, par exemple, un radical méthyl, éthyl, propyl, isopropyl, butyl, isobutyl ou pentyl; le terme radical aralcoyle renfermant de 7 à 12 atomes de carbone désigne, par exemple, un radical benzyl ou phénéthyl, le terme radical cycloalcoylalcoyle renfermant de 4 à 7 atomes de carbone désigne, par exemple, un radical cyclopropylméthyl; le terme radical alcényle renfermant de 3 à 7 atomes de carbone désigne, par exemple, un radical allyl ou butèn-2-yl; le terme radical alcynyle renfermant de 3 à 7 atomes de carbone désigne, par exemple, un radical propargyl.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

La liaison portée en pointillé signifie que la jonction entre le cycle morpholino et le cycle cycloheptène est trans.

Bien entendu, les dérivés racémiques de formule I, aussi bien que les isomères optiquement actifs, entrent dans le cadre de l'invention.

Parmi les produits objet de l'invention, on peut citer notamment les dérivés répondant à la formule I ci-dessus, ainsi que leurs sels d'addition avec les acides minéraux ou organiques, caractérisés en ce que, dans ladite formule I, R représente un atome d'hydrogène, un radical méthyl ou un radical benzyle, $R_1$ est tel que défini précédemment et $R_2$ représente un atome d'hydrogène ou un radical alcoyle renfermant de 1 à 5 atomes de carbone ou cycloalcoylalcoyle renfermant de 4 à 7 atomes de carbone.

Parmi ceux-ci, on peut citer plus particulièrement les dérivés caractérisés en ce que, dans ladite formule I, R représente un atome d'hydrogène, $R_1$ représente un atome d'hydrogène ou de brome, et $R_2$ représente un atome d'hydrogène ou un radical alcoyle renfermant de 1 à 5 atomes de carbone, ainsi que leurs sels d'addition avec les acides minéraux ou organiques, et tout particulièrement les dérivés dans les exemples.

L'invention a également pour objet un procédé de préparation des dérivés, tels que définis par la formule I ci-dessus, ainsi que de leurs sels, caractérisé en ce que l'on fait réagir un produit de

formule II:

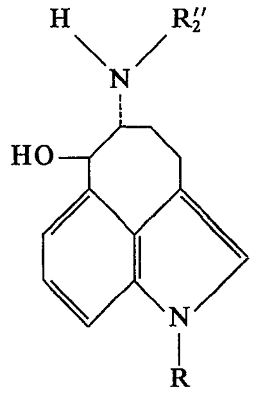

(II)

dans laquelle R à la signification déjà indiquée et $R_2''$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, avec le chlorure de chloroacétyle, pour obtenir un produit de formule III:

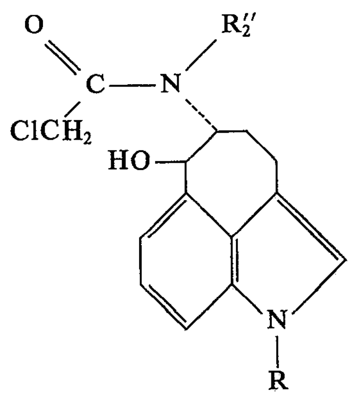

(III)

dans laquelle R et $R_2''$ ont la signification déjà indiquée, que l'on cyclise pour obtenir un produit de formule IV:

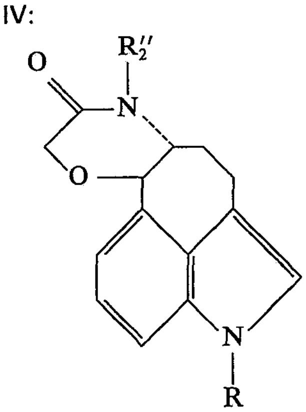

(IV)

dans laquelle R et $R_2''$ ont la signification déjà indiquée, que l'on réduit pour obtenir un produit de formule $I_A$:

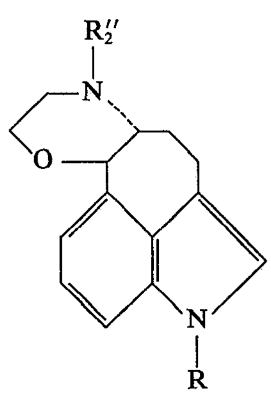

($I_A$)

dans laquelle R et $R_2''$ ont la signification déjà indiquée, correspondant à un produit de formule I, dans laquelle $R_2$ a les valeurs de $R_2''$ et $R_1$ représente un atome d'hydrogène, produit de formule $I_A$, que:
— soit l'on isole et, si désiré, salifie,
— soit l'on traite le produit de formule $I_A$ à l'aide d'un agent d'halogénation, pour obtenir un produit de formule $I_B$:

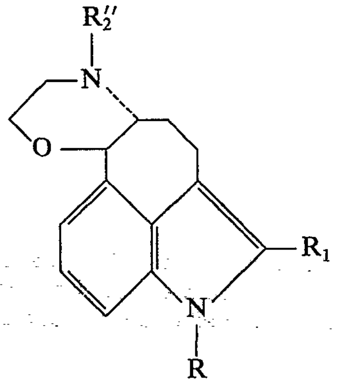

($I_B$)

dans laquelle R et $R_2''$ ont la significtion déjà indiquée et $R_1'$ représente un atome de chlore ou de brome, correspondant à un produit de formule I, dans laquelle $R_1$ a la valeur de $R_1'$ et $R_2$ a la valeur de $R_2''$, produit de formule $I_B$, que l'on isole et, si désiré, salifie:
— soit, dans le cas où $R_2''$ représente un atome d'hydrogène, l'on traite le produit de formule $I_A$ par un halogénure de formule V:

$$Hal—R_2' \qquad (V)$$

dans laquelle Hal représente un atome de chlore, de brome ou d'iode, et $R_2'$ a la signification de $R_2$ déjà indiquée, à l'exception de l'hydrogène, pour obtenir un produit de formule $I_C$:

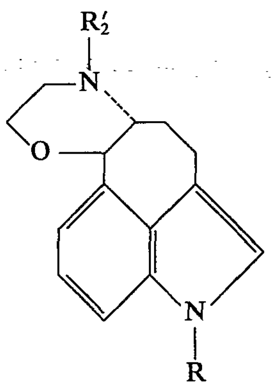

($I_C$)

dans laquelle R et $R_2'$ ont la signification déjà indiquée, correspondant à un produit de formule I dans laquelle $R_2$ a les valeurs de $R_2'$ et $R_1$ représente un atome d'hydrogène, produit de formule $I_C$, que ou bien l'on isole et, si désiré, salifie, ou bien l'on traite à l'aide d'un agent d'halogenation, pour obtenir un produit de formule $I_D$:

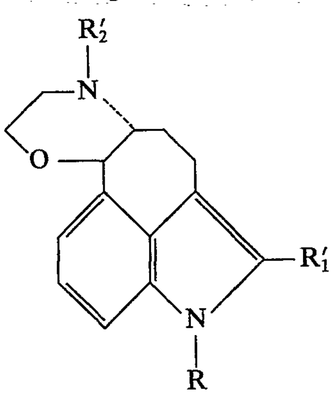

($I_D$)

dans laquelle R, $R_1'$ et $R_2'$ ont la signification déjà indiquée, correspondant à un produit de formule I dans laquelle $R_1$ a les valeurs de $R_1'$ et $R_2$ a les valeurs de $R_2'$, produit de formule $I_D$, que l'on isole, et, si

désiré, salifie,

— soit, dans le cas où $R_2''$ représente un atome d'hydrogène, l'on traite le produit de formule $I_A$ par un agent de formylation, pour obtenir un produit de formule VI:

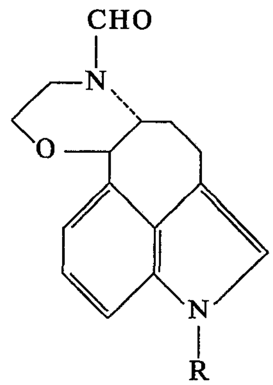

(VI)

dans laquelle R a la significtion déjà indiquée, que l'on traite à l'aide d'un agent réducteur pour obtenir un produit de formule $I_E$:

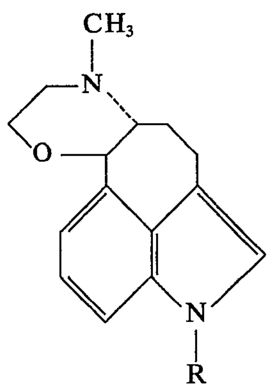

($I_E$)

dans laquelle R a la signification déjà indiquée, correspondant à un produit de formule I, dans laquelle $R_1$ représente un atome d'hydrogène et $R_2$ représente un radical méthyle, produit de formule $I_E$, que:
ou bien l'on isole et, si désiré, salifie,
ou bien l'on traite à l'aide d'un agent d'halogénation pour obtenir un produit de formule $I_F$:

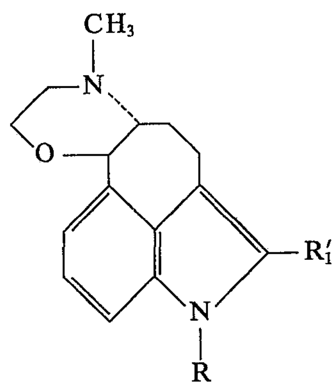

($I_F$)

dans laquelle R et $R_1'$ ont la signification déjà indiquée, correspondant à un produit de formule I, dans laquelle $R_1$ a les valeurs de $R_1'$ et $R_2$ représente un radical méthyle, produit de formule $I_F$ que l'on isole et, si désiré, salifie,

— soit, dans le cas où R représente un atome d'hydrogène et $R_2''$ a les valeurs telles que définies précédemment à l'exception d'hydrogène, l'on traite le produit de formule $I_A$ par un halogénure de formule

Hal—R'

dans laquelle Hal représente un atome de chlore, de brome ou d'iode et R' a la signification de R, à

l'exception d'hydrogène, pour obtenir un produit de formule $I_G$:

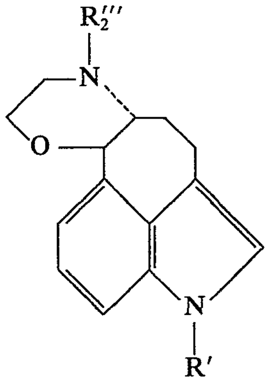

$(I_G)$

dans laquelle R' a la signification déjà indiquée, et $R_2'''$ a la signification de $R_2''$, à l'exception d'hydrogène, correspondant à un produit de formule I, dans laquelle R a les valeurs de R' et $R_2$ a les valeurs de $R_2'''$, produit de formule $I_G$ que ou bien l'on isole et, si désiré, salifie, ou bien l'on traite par un agent d'halogénation, pour obtenir un produit de formule $I_H$:

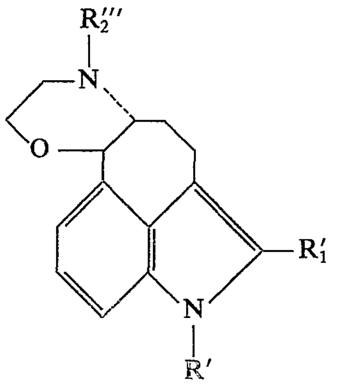

$(I_H)$

dans laquelle R', $R_1'$ et $R_2'''$ ont la signification déjà indiquée, correspondant à un produit de formule I, dans laquelle R a les valeurs de R', $R_1$ celles de $R_1'$ et $R_2$ celles de $R_2'''$, produit de formule $I_H$ que l'on isole et, si désiré, salifie.

La condensation du produit de formule II avec le chlorure de chloro acétyle est avantageusement effectuée en présence d'un agent fixateur d'acide, de préférence la soude, dans un solvant organique tel que le dioxanne ou le tétrahydrofuranne, mais de préférence ce dernier, en présence d'eau.

La cyclisation du produit de formule III est effectuée de préférence à l'aide d'hydrure de sodium, mais on peut utiliser d'autres bases telles que la soude ou la potasse; elle est avantageusement réalisée dans le diméthylformamide ou le diméthoxyéthane.

La réduction du produit de formule IV est réalisée de préférence à l'aide d'hydrure d'aluminium-lithium. Pour mener à bien cette réduction on peut utiliser un solvant tel que le dioxanne, et, de préférence, le tétrahydrofuranne.

L'agent d'halogénation peut être, par exemple, la N-chloro succinimide, dans le cas de la chloration, la N-bromo succinmmide, ou de préférence le complexe bromé dela 2-pyrrolidone de formule:

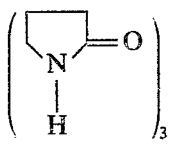 HBr $Br_2$

dans le cas de la bromation.

L'halogénure de formule V est de préférence un iodure; il réagit avantageusement avec l'amine secondaire de formule $I_A$ en présence d'un agent fixateur d'acide, comme, par exemple, un carbonate alcalin tel que le carbonate de potassium, dans un solvant tel que le diméthylformamide.

La formylation peut être réalisée à l'aide d'acide formique mais on utilise de préférence l'anhydride formylacétique; ce dernier réactif est avantageusement utilisé dans un solvant tel que le tétrahydrofuranne.

L'halogénure de formule Hal—R' est de préférence un iodure et l'on opère en milieu fortement basique, par exemple en présence d'amidure de sodium.

Les racémates de formule I peuvent être dédoublés en leurs énantiomères optiquement actifs par des méthodes connues en elles-mêmes, telles que, par exemple, la formation de sels au moyen d'acides optiquement actifs.

Les dérivés de formule I présentent un caractère basique. On peut avantageusement préparer les sels d'addition des dérivés de formule I, en faisant réagir, en proportions sensiblement stoechiométriques, un acide minéral ou organique avec lesdits dérivés de formule I. Les sels peuvent être préparés sans isoler les bases correspondantes.

Les dérivés, objet de la présente invention, possèdent de très intéressantes propriétés pharmacologiques; ils sont doués notamment de remarquables propriétés agonistes dopaminergiques et anti-anoxiques. De plus, certains dérivés de formule I possédent, en outre, des propriétés anti-ulcères.

Ces propriétés sont illustrées plus loin dans la partie expérimentale.

Ces propriétés justifient l'utilistion des dérivés du cycloheptindole, ainsi que de leurs sels pharmaceutiquement acceptables, à titre de médicaments.

La présente demande a ainsi également pour objet l'application, à titre de médicaments, des dérivés du cycloheptindole, tels que définis par la formule I, ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés du cycloheptindole répondant à la formule I, dans laquelle R représente un atome d'hydrogène ou un radical benzyl, $R_1$ est tel que défini précédemment et $R_2$ représente un atome d'hydrogène ou un radical alcoyle renfermant de 1 à 5 atomes de carbone ou cycloalcoylalcoyle renfermant de 4 à 7 atomes de carbone, ainsi que par leurs sels d'addition avec les acides pharmaceutiquement acceptables.

Parmi ceux-ci, on retient notamment ceux répondant à la formule I, dans laquelle R représente un atome d'hydrogène, $R_1$ représente un atome d'hydrogène ou de brome, et $R_2$ représente un atome d'hydrogène ou un radical alcoyle renfermant de 1 à 5 atomes de carbone, ainsi que leurs sels d'addition avec les acides pharmaceutiquement acceptables.

Parmi ces derniers, on retient tout particulièrement, les dérivés cités dans les exemples.

Les médicaments selon l'invention trouvent leur emploi, par exemple, dans le traitement des syndromes neurologiques d'origine extrapyramidale, par exemple dans le traitement de la maladie de Parkinson et dans le traitement des syndromes parkinsonniens post-encéphalitiques; ils peuvent être également utilisés dans le traitement de l'hypersécrétion de prolactine par l'antéhypophyse, par exemple dans le traitement de l'hypogonadisme de la femme ou de l'homme. Ils peuvent être aussi utilisés dans le traitement de la sénescence cérébrale ou des manifestations d'une hypoxie cérébrale.

Certains médicaments selon l'invention peuvent en outre être utilisés notamment pour le traitement des hyperchlorhydries, des ulcères gastriques et des affections gastroduodénales s'accompagnant d'hyperacidité gastrique.

La dose usuelle, variable selon le produit utilisé, le sujet traité et l'affection en cause peut-être, par exemple, de 5 mg à 100 mg par jour par voie orale chez l'homme avec le dérivé de l'exemple 3 pour le traitement de la maladie de Parkinson.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les dérivés de formule II, telle que définie précédemment, sont des produits nouveaux. Les dérivés de formule II dans laquelle $R_2''$ représente un radical alcoyle renfermant de 1 à 5 atomes de carbone, cycloalcoylalcoyle renfermant de 4 à 7 atomes de carbone, ou aralcoyle renfermant de 7 à 12 atomes de carbone éventuellement substitué, peuvent être préparés de façon générale en faisant réagier un produit de formule II dans laquelle $R_2''$ représente un atome d'hydrogène, avec l'anhydride trifluoroacétique, de préférence en présence d'une base, pour obtenir un produit de formule VII:

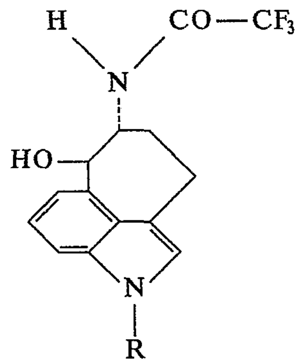

(VII)

dans laquelle R a la signification déjà indiquée, que l'on fait réagir en présence d'un agent basique tel qu'un carbonate ou un hydroxyde alcalin, ou une amine tertiaire, avec un halogénure de formule VIII:

$$Hal-R_2''$$ (VIII)

dans laquelle Hal et $R_2''$ ont la signification déjà indiquée, pour obtenir un produit de formule IX:

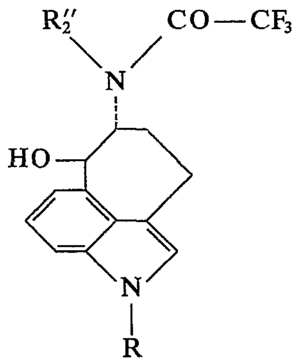

(IX)

dans laquelle R et $R_2''$ ont la signification déjà indiquée, que l'on soumet à un agent capable d'hydrolyser l'amide trifluoroacétique pour obtenir le produit de formule II cherché.

Pour la préparation des produits de formule II, il peut être intéressant dans certains cas d'opérer de façon particulière:

— Pour obtenir un produit de formule II dans laquelle $R_2''$ représente un radical méthyl, on peut préparer un carbamate d'alcoyle, de préférence d'éthyle, sur l'atome d'azote non indolique, puis réduire ledit carbamate; le carbamate peut être préparé par action sur le produit de formule II dans laquelle $R_2''$ représente un atome d'hydrogène, d'un haloformiate d'alcoyle en présence d'une base telle qu'un carbonate alcalin; la réduction est par exemple effectuée à l'aide d'hydrure d'aluminium lithium au reflux du tétrahydrofuranne. Un exemple d'une telle préparation figure ci après dans la partie expérimentale.
— Pour préparer un produit de formule II dans laquelle $R_2''$ représente un radical éthyl, on peut préparer le dérivé N-acétylé correspondant puis le réduire.
— Pour préparer un produit de formule II dans laquelle $R_2''$ représente un radical isopropyl, on peut faire réagir un produit de formule II dans laquelle $R_2''$ représente un atome d'hydrogène, avec l'acétone en présence d'un réducteur tel que le cyanaoborohydrure de sodium. Un exemple d'une telle préparation figure ci-après dans la partie expérimentale.

Le produit de formule II dans laquelle R et $R_2''$ représentent un atome d'hydrogène peut être préparé en traitant la 3,4-dihydro 1H-cyclohept [c,d] indol-6-one décrite dans Chem. Pharm. Bull. 25 (11), p. 3023, 1977, par du nitrite de tert-butyle en présence d'acide chlorhdrique, puis en réduisant le céto-oxime obtenu, par exemple par hydrogénation catalytique, suivie de l'action du borohydrure de sodium. Un exemple d'une telle préparation figure ci-après dans la partie expérimentale.

Les produits de formule II dans laquelle $R_2''$ représente un atome d'hydrogène et R a la signification déjà indiquée, peuvent être préparés en faisant réagir un halogénure d formule X:

$$Hal-R$$ (X)

dans laquelle Hal et R ont la signification déjà indiquée, R étant différent d'hydrogène, sur le 3,4-dihy-

dro 1H-cyclohept [c,d] indol-6-one, par transfert de phase, selon la technique de A. Bosco et al, décrite dans Synthesis 2, 124 (1976), par exemple en présence d'un sel d'ammonium quaternaire, tel que le chlorure de tétrabutyl ammonium, le chlorure de benzyltriphénylphosphonium, ou de préférence l'hydrogénosulfate de tétrabutyl ammonium, de soude aqueuse et de benzène, puis préparation de l'oxime et réduction comme indiqué ci-dessus.

Il va être donné maintenant, à titre non limitatif, des exemples de mise en oeuvre de l'invention.

## Exemple 1

### Chlorhydrate de (7a RS-trans) 4,6,7,7a,8,9,10,11a-octahydro [1,4]-oxazino [2',3'-4,5] cyclohept [1,2,3-c,d] indole

### Stade A

### (5RS-trans) 2-chloro N-(6-hydroxy 3,4,5,6-tétrahydro 1H-cyclohept [c,d] indol-5-yl)acétamide

On dissout 2,47 g de soude dans 25 $cm^3$ d'eau, ajoute sous atmosphère inerte 9,8 g de (RS trans) 5-amino 3,4,5,6-tétrahydro 1H-cyclohept [c,d] indol-6-ol en solution dans 100 $cm^3$ de tétrahydrofuranne, puis 3,65 $cm^3$ de chlorure de chloroacétyle en 15 minutes sous agitation, agite pendant 45 minutes, décante, lave à l'eau salée, extrait à l'acétate d'éthyle, sèche, amène à sec et obtient 13 g du produit brut cherché.

### Stade B

### (7a RS-trans) 4,6,7,7a,10,11a-hexahydro [1,4]-oxazino [2',3'-4,5] cyclohept [1,2,3-c,d] indol-9 (8H)-one

On lave 2,17 g d'hydrure de sodium en suspension huileuse à 50%, à l'éther de pétrole (Eb = 60—80° C). On y ajoute 10 $cm^3$ de diméthylformamide, introduit le produit obtenu au stade précédent, en solution dans 50 $cm^3$ de dimméthylformamide, en 15 minutes sous atmosphère inerte, agite pendant 1 heure 20 minutes, glace, ajoute 250 $cm^3$ d'eau, agite pendant 30 minutes, essore, lave à l'eau, séche à 80° C sous pression réduite et récupère 9 g d'un solide beige fondant avec décomposition à plus de 280° C.

### Stade C

### Chlorhydrate de (7aRS-trans) 4,6,7,7a,8,9,10,11a-octahydro [1,4]-oxazino [2',3'-4,5] cyclohept [1,2,3-c,d] indole

On met en suspension 6 g d'hydrure d'aluminium-lithium dans 400 $cm^3$ de tétrahydrofuranne, sous atmosphère inerte, introduit en 10 minutes, 12,13 g de produit tel qu'obtenu au stade précédent, porte au reflux pendant 3 heures, introduit à 20° C 6 g d'hydrure d'aluminium-lithium, porte à nouveau au reflux pendant 3 heures 15 minutes, refroidit rapidement à 20° C, introduit 300 $cm^3$ de tétrahydrofuranne hydraté à 20%, agite pendant 15 minutes, filtre, lave avec 1000 $cm^3$ de chlorure de méthylène à 10% de méthanol, amène à sec, et récupère 14 g de produit brut. On reprend au chlorure de méthylène, lave à l'eau, sèche, traite au charbon actif, filtre, amène à sec, purifie par chromatographie sur colonne de silice (éluant: cyclohexane-éthanol-triéthylammine 7-2-1), et obtient 5,61 g d'un solide beige fondant à 198° C puis 204° C.

### Préparation du chlorhydrate

On dissout la base obtenue ci-dessus dans 80 $cm^3$ de méthanol à 60° C, ajoute un excès d'une solution d'acide chlorhydrique dans l'acétate d'éthyle, refroidit, essore, lave à l'acétate d'éthyle, sèche, recristallise dans le méthanol et obtient 6,1 g du produit pur cherché, sous forme d'un solide beige fondant à plus de 280° C avec décomposition.

Analyse: $C_{14}H_{16}N_2O$, HCl = 264,75

| | | | | |
|---|---|---|---|---|
| Calculé: | C 63,52 | H 6,47 | N 10,58 | Cl 13,39% |
| Trouvé: | C 63,6 | H 6,5 | N 10,3 | Cl 13,4% |

Le (5-RS trans) amino 3,4,5,6-tétrahydro 1H-cyclohept [c,d] indol-6-ol de départ peut être préparé comme suit:

a) Préparation de la 5-oxime de 3,4-dihydro 1H-cyclohept [c,d] indole 5,6-dione

On dissout sous agitation et azote 18,5 g de 3,4-dihydro 1H-cyclohept [c,d] indol-6-one décrite dans Chem. Pharm. Bull. 25 (11) p. 3023, 1977, dans 185 $cm^3$ de tétrahydrofuranne, refroidit au bain de glace, ajoute 22 $cm^3$ d'une solution 5M d'acide chlorhydrique dans l'éthanol, puis 13,5 $cm^3$ de nitrite de tert butyle, agite encore pendant 30 minutes au bain de glace, essore, lave à l'éther, sèche sous pression réduite à 80° C et obtient 19,4 g de cristaux fondant avec décomposition à 235° C.

b) Préparation du (5-RS trans) amino 3,4,5,6 tétrahydro 1H-cyclohept [c,d] indol-6-ol

On met en suspension 5 g du produit obtenu au stade précédent dans 100 $cm^3$ de méthanol, ajoute 2,5 g de palladium sur charbon actif à 10%, agite pendant 40 minutes à température ambiante sous hydrogène, filtre, refroidit le filtrat au bain de glace, ajoute 2,5 g de borohydrure de sodium en 10 minutes, laisse revenir à température ambiante sous atmosphère inerte, agite pendant 1 heure, évapore à sec, reprend au chlorure de méthylène au reflux, filtre, distille à sec, redissout à 50° C dans 7 $cm^3$ de méthanol, ajoute 70 $cm^3$ d'acétate d'éthyle, filtre, rince à l'acétate d'éthyle, concentre à 50 $cm^3$, ajoute alors 25 $cm^3$ de solution d'acide acétique dans l'acétate d'éthyle 1,1N, laisse reposer à température ambiante pendant 1 heure, glace pendant 30 minutes, essore, sèche sous pression réduite à 70° C, et récupère 4,8 g de l'acétate du produit attendu fondant à 150° C.

Par recristallisation dans le mélange méthanol-acétate d'éthyle 1-1, l'acétate est un solide blanc cristallisé fondant à 165° C.

Analyse de l'acétate: $C_{12}H_{14}N_2O$, $C_2H_4O_2$ = 262,30

| | | | |
|---|---|---|---|
| Calculé: | C 64,10 | H 6,92 | N 10,68% |
| Trouvé: | C 64,2 | H 7,1 | N 10,4% |

Libération de la base

On met en suspension 10,8 g d'acétate tel qu'obtenu ci-dessus dans 300 $cm^3$ d'acétate d'éthyle, ajoute sous agitation 100 $cm^3$ d'ammoniaque concentrée, sature de chlorure de sodium, agite sous azote pendant 15 minutes, décante, lave à l'eau salée, sèche, amène à sec et obtient 9,8 g du produit attendu.

## Exemple 2

Chlorhydrate du (7aRS-trans) 8-méthyl 4,6,7,7a,8,9,10,11a-octahydro [1,4] oxazino [2',3'-4,5] cyclohept [1,2,3-c,d] indole

### Stade A

(7aRS-trans) 4,6,7,7a,8,9,10,11a octahydro [1,4] oxazino [2',3',4,5] cyclohept [1,2,3-c,d] indol-8-carboxaldéhyde

On met en suspension 4,3 g de la base du produit de l'exemple 1, dans 86 $cm^3$ de tétrahydrofuranne, ajoute sous atmosphère inerte 1,9 $cm^3$ d'anhydride formyl acétique, agite pendant 50 minutes, essore, lave à l'éther éthylique, sèche à 60° C sous pression réduite et obtient 4,65 g du produit cherché sous forme d'un solide blanc fondant vers 275° C.

### Stade B

Chlorhydrate du (7aRS-trans) 8-méthyl 4,6,7,7a,8,9,10,11a octahydro [1,4] oxazino [2',3'-4,5] cyclohept [1,2,3-c,d]indole

On met en suspension sous atmosphère inerte 1,38 g d'hydrure d'aluminium-lithium dans 80 $cm^3$ de tétrahydrofuranne, introduit en 10 minutes à +6, +8° C le produit obtenu au stade précédent, porte au reflux pendant 50 minutes, introduit à 10° C 30 $cm^3$ de tétrahydrofuranne hydraté à 20%, filtre, lave la phase organique à l'eau salée, extrait à l'acétate d'éthyle, sèche, amène à sec et récupère 4,2 g d'un

solide blanc fondant à 170° C correspondant à la base du produit attendu.

Préparation du chlorhydrate

On dissout les 4,2 g de base à 60° C dans 80 cm³ d'acétate d'éthyle, ajoute un excès d'une solution d'acide chlorhyrique dans l'acétate d'éthyle, concentre sous atmosphère inerte à température ambiante, glace pendant 1 heure, essore, lave à l'acétate d'éthyle, sèche, recristallise dans le méthanol et obtient 3,8 g d'un solide blanc fondant vers 270° C.

Analyse: $C_{15}H_{18}N_{20}$, HCl = 278,78

| | | | | |
|---|---|---|---|---|
| Calculé: | C 64,63 | H 6,87 | N 10,05 | Cl 12,72% |
| Trouvé: | C 64,9 | H 6,9 | N 10,0 | Cl 13,0% |

Exemple 3

Chlorhydrate du (7aRS-trans)4,6,7,7a,8,9,10,11a-octahydro 8-propyl [1,4] oxazino [2',3'-4,5] cyclohept-[1,2,3-c,d] indole

On met en suspension sous atmosphère inerte à 60° C pendant 4 heures 5,34 g du produit de 'exemple 1, 8,46 g de carbonate de potassium et 4,03 cm³ d'iodopropane à 98%, dans 50 cm³ de diméthylformamide, ramène à température ambiante, ajoute 300 cm³ d'eau, extrait à l'acétate d'éthyle, lave à l'eau, sèche, amène à sec et obtient 5,5 g de la base du produit cherché fondant à 170° C.

Préparation du chlorhydrate

On dissout la base dans 120 cm³ d'acétate d'éthyle à 50° C, 60° C, ajoute lentement sous agitation 40 cm³ d'une solution d'acide chlorhydrique dans l'acétate d'éthyle environ 2N, cristallise sous agitation à 50° C, laisse 1 heure en réfrigérateur, essore, lave à l'acétate d'éthyle, sèche à 60° C sous pression réduite, traite au charbon actif, recristallise dans le méthanol et obtient 4,35 g d'un solide blanc fondant vers 260° C avec décomposition.

Analyse: $C_{17}H_{22}N_2O$, HCl = 306,83

| | | | | |
|---|---|---|---|---|
| Calculé: | C 66,55 | H 7,55 | Cl 11,55 | N 9,13% |
| Trouvé: | C 66,5 | H 7,5 | Cl 11,4 | N 9,1% |

Exemple 4

Chlorhydrate de (7a RS-trans) 5-bromo 4,6,7,7a,8,9,10,11a-octahydro 8-propyl [1,4] oxazino [2',3'-4,5] cyclohepta [1,2,3-c,d] indole

On dissout 3 g de produit obtenu à l'exemple 3, sous forme de base, dans 100 cm³ de chlorure de méthylène et ajoute lentement 5 g d'hydrotribromure de pyrrolidone en solution dans 250 cm³ de chlorure de méthylène. On agite pendant 30 minutes à 20° C, puis de sodium, décante, lave la phase organique à l'eau, sèche, évapore le solvant et obtient 4,1 g de produit brut que l'on chromatographie sur silice en éluant au mélange cyclohexane-chloroforme-triéthylamine (4-4-2). On obtient 2,6 g de produit attendu.

Préparation du chlorhydrate

On dissout les 2,6 g de produit obtenu ci-dessus dans 3 cm³ d'éthanol et ajoute une solution d'acide chlorhydrique dans l'acétate d'éthyle jusqu'à pH 2. On obtient après cristallisation, essorage puis recristallisation dans l'isopropanol, 1,3 g de produit attendu. F ≃ 265° C.

Analyse: $C_{17}H_{22}BrClN_2O$ = 385,75

| | | | | | |
|---|---|---|---|---|---|
| Calculé: | C 52,93 | H 5,75 | Br 20,72 | Cl 9,19 | N 7,26% |
| Trouvé: | C 53,2 | H 5,8 | Br 21,2 | Cl 8,5 | N 7,3% |

Exemple 5

Chlorhydrate de (7a R,S-trans) 4,6,7,7a,8,9,10,11a-octahydro 4-méthyl 8-propyl [1,4] oxazino [2',3'-4,5] cyclohept-[1,2,3-c,d] indole

On condense 35 $cm^3$ d'ammoniac et ajoute 161 mg de sodium. On agite pendant 15 minutes vers —40° C, ajoute 20 mg de nitrate ferrique, agite pendant 20 minutes vers —40° C, puis ajoute rapidement 1,2 g de produit obtenu à l'exemple 3, sous forme de base, en solution dans 18 $cm^3$ de tétrahydrofuranne. On agite pendant 10 minutes à —40° C, puis ajoute 0,5 $cm^3$ d'iodure de méthyle. On laisse remonter la température, ajoute 150 $cm^3$ d'eau, extrait au chlorure de méthylène, lave à l'aide d'une solution aqueuse saturée de chlorure de sodium, sèche, évapore le solvant et obtient 1,3 g de produit brut que l'on dissout dans l'éther. On traite au charbon actif, évapore le solvant et obtient 1,25 g de produit attendu.

Préparation du chlorhydrate

On dissout les 1,25 g de produit obtenu ci-dessus, dans 5 $cm^3$ d'acétate d'éthyle et ajoute une solution d'acide chlorhydrique dans l'acétate d'éthyle jusqu'à pH 2. On essore les cristaux formés, les lave à l'acétate d'éthyle et à l'éther éthylique puis les recristallise dans un mélange méthanol-isopropanol. On obtient 1 g de produit attendu. F = 240—250° C.

Analyse: $C_{18}H_{25}ClN_2O$ = 320,86

| | | | | |
|---|---|---|---|---|
| Calculé: | C 67,38 | H 7,85 | Cl 11,05 | N 8,73% |
| Trouvé: | C 67,5 | H 7,9 | Cl 11,0 | N 8,6% |

Préparation A

Chlorhydrate de (RS-trans) 5-méthylamino 3,4,5,6-tétrahydro 1H-cyclohept [c,d] indol-6-ol

a) RS-trans N-(6-hydroxy 3,4,5,6-tétrahydro 1H-cyclohept [c,d] indol-5-yl) carbamate d'éthyle

On maintient sous agitation 2,62 g d'acétate de (RS-trans) 5-amino 3,4,5,6-tétrahydro 1H-cyclohept [c,d] indol-6-ol, 52 $cm^3$ d'acétate d'éthyle, 40 $cm^3$ d'eau et 2,62 g de carbonate de potassium, ajoute en 10 minutes à température ambiante 2,6 $cm^3$ de chloroformiate d'éthyle, agite pendant 1 heure, distille sous pression réduite, dilue avec un peu d'eau glacée, triture pendant 30 minutes à température ambiante, essore et sèche sous pression réduite à 60° —70° C. On introduit le produit dans du méthanol, agite, filtre, concentre, amorce la cristallisation, glace pendant 3 heures et récupère 1,45 g de cristaux fondant à 190° C.

b) Chlorhydrate de RS-trans 5-méthylamino 3,4,5,6-tétrahydro 1H-cyclohept [c,d] indol-6-ol

On met en suspension à température ambiante sous atmosphère inerte et sous agitation 3,6 g d'hydrure d'aluminium dans 50 $cm^3$ de tétrahydrofuranne, ajoute en 20 minutes une solution de 1,83 g de carbamate tel qu'obtenu ci-dessus dans 75 $cm^3$ de dioxanne. On porte au reflux pendant 2 heures, refroidit, ajoute en 30 minutes 50 $cm^3$ de tétrahydrofuranne hydraté à 10%, dilue par 50 $cm^3$ d'eau, filtre, rince le filtre à l'acétate d'éthyle, ajoute du chlorure de sodium au filtrat, décante, extrait à l'acétate d'éthyle, lave à l'eau puis à l'aide d'une solution aqueuse saturée de chlorure de sodium, sèche sur sulfate de sodium, distille à sec, et récupère 2 g de produit brut.

Préparation du chlorhydrate

On dissout le produit obtenu ci-dessus dans 3 $cm^3$ d'éthanol, ajoute goutte à goutte 1,5 $cm^3$ d'une solution d'acide chlorhydrique dans l'éthanol 5N, glace pendant 1 heure, essore, lave à l'éthanol, sèche sous pression réduite à 70° C et obtient 1,24 g de cristaux fondant à 224° C avec décomposition.

Analyse: $C_{13}H_{16}N_2O$, HCl = 252,7

| | | | | |
|---|---|---|---|---|
| Calculé: | C 61,78 | H 6,78 | N 11,08 | Cl 14,03% |
| Trouvé: | C 61,8 | H 6,7 | N 11,1 | Cl 13,9% |

Préparation B

RS-trans 5-(1-méthyléthyl) amino 3,4,5,6-tétrahydro 1H-cyclohept [c,d] indol-6-ol

On met en suspension sous agitation et atmosphères inerte 10,5 g d'acétate de (RS-trans) 5-amino 3,4,5,6-tétrahydro 1H-cyclohept [c,d] indol-6-ol dans 105 $cm^3$ de méthanol et 52,3 $cm^3$ d'acétone, refroidit vers 0°C, ajoute en 15 minutes 10,5 g de cyanoborohydrure de sodium, agite pendant 3 heures 30 minutes vers 0°C, filtre, rince à l'acétate d'éthyle, évapore à sec, chromatographie sur colonne de silice sous pression (éluant benzène-acétate d'éthyle-triéthylamine 7-2-1) et récupère 6,32 g de cristaux. On redissout les cristaux dans 6 $cm^3$ d'acétate d'éthyle à chaud, laisse refroidir, amorce la cristallisation à —15°—20°C, laisse à cette température pendant 1 heure 30 minutes, essore, sèche sous pression réduite à température ambiante et obtient 4,03 g de cristaux blancs fondant à 100°C.

Analyse: $C_{15}H_{20}N_2O = 244,338$

| | | | |
|---|---|---|---|
| Calculé: | C 73,74 | H 8,25 | N 11,46% |
| Trouvé: | C 73,5 | H 8,3 | N 11,4% |

Exemple 6

On a préparé des comprimés répondant à la formule:

— Chlorhydrate de (7a RS-trans) 8-méthyl 4,6,7,7a,8,9,10,11a-octahydro [1,4]-oxazino [2',3'-4,5] cyclohept [1,2,3-c,d] indole 20 mg
— Excipient q.s. pour un comprimé terminé à 100 mg
(Détail de l'excipient: lactose, amidon, talc, stéarate de magnésium).

Exemple 7

On a préparé des comprimés répondant à la formule:

— Chlorhydrate de (7a RS-trans) 4,6,7,7a,8,9,10,11a octahydro 8-propyl [1,4] oxazino [2',3'-4,5] cyclohept [1,2,3-c,d] indole 10 mg
— Excipient q.s. pour un comprimé terminé à 100 mg
(Détail de l'excipient: lactose, amidon, talc, stéarate de magnésium).

Etude pharmacologique

1) Comportement de rotation après lésion unilatérale du faisceau nigrostriatal par la 6-hydroxydopamine

Technique

La lésion est effectuée chez des rats mâles de 220 g environ par injection unilatérale dans le faisceau dopaminergique nigrostrié, de 8 µg de 6-hydroxydopamine en solution à 2 µg/µl (U. Ungerstedt, Acta Physiol. Scand. 1971, 82, suppl. 367, 69—93).

Chez de tels animaux, les agonistes dopaminergiques directs, tels que l'apomorphine, administrés par voie générale, entraînent un comportement de rotation dans la direction contralatérale au côté lésé.

Le composé étudié est administré au moins 5 semaines après la lésion. Les animaux sont placés dans un rotomètre automatisé qui permet de compter le nombre de rotations effectuées par chaque animal dans les deux sens.

Les produits des exemples 1, 2 et 3 ont été administrés par la voie intrapéritonéale. Le produit de l'exemple 1 a provoqué $337 \pm 99$ rotations contralatérales à la dose de 5 mg/kg, le produit de l'exemple 2: $364 \pm 50$ rotations contralatérales à la dose de 1 mg/kg, et le produit de l'exemple 3: $657 \pm 205$ rotations contralatérales à la dose de 0,1 mg/kg.

2) Affinités pour les récepteurs dopaminergiques

On homogénéise au vingtième (poids/volume) dans le sucrose 0,32 M, les corps striés prélevés des cerveaux de 6 rats mâles pesant 150 g en moyenne. Après centrifugation du mélange homogénéisé, à

1000 g pendant 10 minutes à 0°C, le surnageant est centrifugé à 30 000 g pendant 15 minutes à +4°C. Le culot est repris dans 25 ml de tampon Tris HCl 50 mM pH 7,7 et centrifugé à 30 000 g pendant 15 minutes à +4°C. Le nouveau culot est repris dans 50 ml de tampon Krebs Tris HCl pH 7,3 et la suspension est préincubée pendant 10 minutes à 37°C. On fait ensuite incuber pendant 20 minutes au bainmarie à +37°C en présence de spiropéridol $3_H$, seul ou avec un excès d'halopéridol, ou avec des concentrations croissantes du produit à tester. Les suspension incubées sont filtrées sur Whatman GF/C et les filtres sont lavés par trois fois 5 ml de tampon Tris HCl 50 mM. La radioactivité des filtres est mesurée par scintillation liquide.

On établit la courbe du pourcentage de fixation du spiropéridol $3_H$ en fonction dela concentration du produit à tester.

On appelle concentration inhibitrice 50 la concentration du produit à tester qui inhibe de 50% la fixation maximale du spiropéridol $3_H$.

On a obtenu les résultats suivants:

| Produits testés | Concentration inhibitrice 50 en nano-moles |
|---|---|
| Exemple 1 | 10 000 |
| Exemple 2 | 950 |
| Exemple 3 | 270 |

Les produits des exemples 1, 2 et 3 se fixent donc sur les récepteurs dopaminergiques.

3) Etude de l'anoxie hypobare chez la souris

On utilise des souris mâles d'un poids de 20 à 22 g, à jeun depuis 5 heures, réparties par groupes de 10 animaux. On détermine le temps de survie des souris placées dans une enceinte hermétique dans laquelle on amène la pression à 90 mm de mercure au moyen d'une pompe. Les produits étudiés sont administrés par voie orale, trente minutes avant l'essai. Les témoins ne recoivent aucun produit. L'observation est effectuée pendant 2 minutes.

Les résultats suivants correspondent à l'augmentation du temps de survie exprimés en pourcentage par rapport aux animaux témoins:

Produit de l'exemple 1

| | |
|---|---|
| 50 mg/kg | 145% |
| 10 mg/kg | 51% |
| 2 mg/kg | 14% |

Produit de l'exemple 2

| | |
|---|---|
| 25 mg/kg | 64% |
| 5 mg/kg | 30% |

Produit de l'exemple 3

| | |
|---|---|
| 25 mg/kg | 86% |
| 5 mg/kg | 102% |
| 1 mg/kg | 3% |

4) Détermination del'activité antiulcéreuse

La technique utilisée est décrite par SHAY et al dans Gastroenterology, 5, 43 (1945).

La technique de SHAY consiste à induire chez des rats des ulcères au niveau de l'estomac, par ligature du pylore.

Les animaux sont anesthésiés à l'éther. Une incision longitudinale est faite 1 cm environ au dessous du sternum, la partie glandulaire de l'estomac et le duodénum sont mis à jour, et une ligature est posée

quelques mm au dessous du pylore. Le plan musculaire est laissé tel quel et la peau est suturée par 2 agrafes.

Les animaux reçoivent aussitôt après, le dispersif ou la substance à étudier, par voie buccale, sous un volume de 0,5 ml/100 g, et sont maintenus sans nourriture ni boisson jusqu'au sacrifice par saignée carotidienne qui a lieu environ 16 heures après le traitement.

Avant de prélever l'estomac une ligature est posée au-dessus du cardia.

Le liquide gastrique est recueilli afin d'en mesurer le pH.

L'estomac est ensuite ouvert selon la grande courbure, rincé dans le sérum physiologique, et étalé sur du papier millimétrique pour être examiné sous la loupe binoculaire.

On évalue macroscopiquement la gravité des lésions qui est côtée de 0 à 4 pour chaque estomac.

On détermine pour chaque lot de rat, l'intensité moyenne des ulcérations et on calcule la protection en rapportant l'index moyen du groupe à l'index moyen du groupe témoin.

On a obtenu les résultats suivants:

| Produits de l'exemple | Dose (mg/kg) | Ulcération % de protection par rapport aux témoins |
|---|---|---|
| 3 | 25 | 73% |
| | 5 | 51% |

5) Etude de la toxicité aiguë

On a évalué la dose létale $DL_0$ des dérivés des exemples 1, 2 et 3 testés après administration par voie orale chez la souris.

On appelle $DL_0$ la dose maximale ne provoquant aucune mortalité.

Les résultats obtenus sont les suivants:

| Produit de l'exemple | $DL_0$ en mg/kg |
|---|---|
| 1 | 200 |
| 2 | 100 |
| 3 | 100 |
| 4 | >1 000 |
| 5 | 200 |

**Revendications pour les Etats contractants: BE, CH, DE, FR, GB, IT, LI, LU, NL, SE**

1. Nouveaux dérivés du cycloheptindole, ainsi que leurs sels d'addition avec les acides minéraux ou organiques, caractérisés en ce qu'ils répondent à la formule générale I:

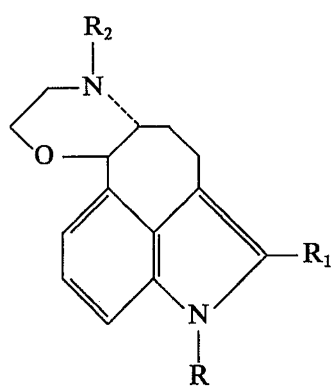

(I)

dans laquelle R représente un atome d'hydrogène, un radical alcoyle renfermant de 1 à 5 atomes de

carbone, ou un radical aralcoyle renfermant de 7 à 12 atomes de carbone, éventuellement substitué par des radicaux chloro, bromo, méthyl, éthyl, méthoxy, trifluorométhyl ou méthylthio, $R_1$ représente un atome d'hydrogène, de chlore ou de brome, et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone ou aralcoyle renfermant de 7 à 12 atomes de carbone, éventuellement substitué par des radicaux chloro, bromo, méthyl, éthyl, méthoxy, trifluorométhyl ou méthylthio.

2. Nouveaux dérivés du cycloheptindole selon la revendication 1, ainsi que leurs sels d'addition avec les acides minéraux ou organiques, caractérisés en ce que R représente un atome d'hydrogène, un radical méthyle ou un radical benzyl, $R_1$ est tel que défini à la revendication 1 et $R_2$ représente un atome d'hydrogène ou un radical alcoyle renfermant de 1 à 5 atomes de carbone ou cycloalcoylalcoyle renfermant de 4 à 7 atomes de carbone.

3. Nouveaux dérivés du cycloheptindole, selon la renvendication 1, ainsi que leurs sels d'addition avec les acides minéraux ou organiques, caractérisés en ce que R représente un atome d'hydrogène, $R_1$ représente un atome d'hydrogène ou de brome, et $R_2$ représente un atome d'hydrogène ou un radical alcoyle renfermant de 1 à 5 atomes de carbone.

4. Le dérivé de formule I telle que définie à la revendication 1 dont le nom suit:

— le (7a RS-trans) 8-méthyl 4,6,7,7a,8,9,10,11a-octahydro [1,4]-oxazino [2',3'-4,5] cyclohept [1,2,3-c,d] indole,

ainsi que ses sels d'addition avec les acides minéraux ou organiques.

5. Procédé de préparation des dérivés de formule I, tels que définis à la revendication 1, ainsi que de leurs sels d'addition avec les acides minéraux ou organiques, caractérisé en ce que l'on fait réagir un produit de formule II:

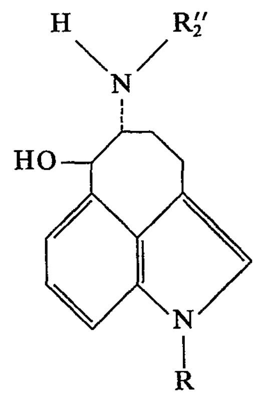

(II)

dans laquelle R a la signification déjà indiquée et $R_2''$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, avec le chlorure de chloroacétyle, pour obtenir un produit de formule III:

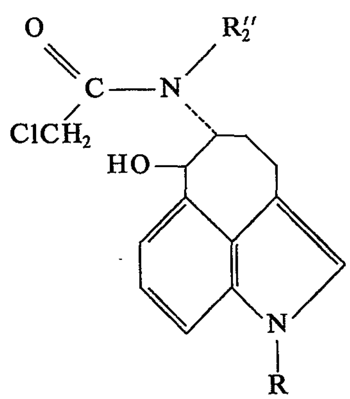

(III)

dans laquelle R et $R_2''$ ont la signification déjà indiquée, que l'on cyclise pour obtenir un produit de

formule IV:

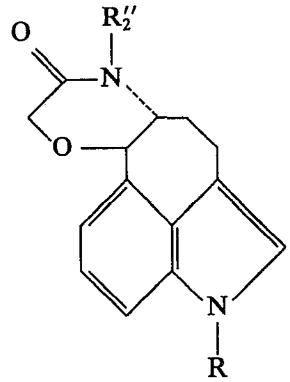

(IV)

dans laquelle R et $R_2''$ ont la signification déjà indiquée, que l'on réduit pour obtenir un produit de formule $I_A$:

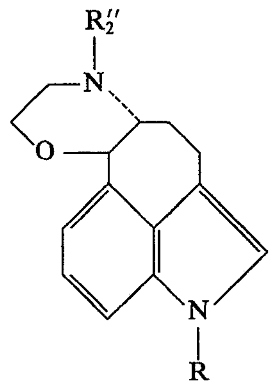

($I_A$)

dans laquelle R et $R_2''$ ont la signification déjà indiquée, correspondant à un produit de formule I, dans laquelle $R_2$ a les valeurs de $R_2''$ et $R_1$ représente un atome d'hydrogène, produit de formule $I_A$, que:

— soit l'on isole et, si désiré, salifie,

— soit l'on traite le produit de formule $I_A$ à l'aide d'un agent d'halogénation, pour obtenir un produit de formule $I_B$:

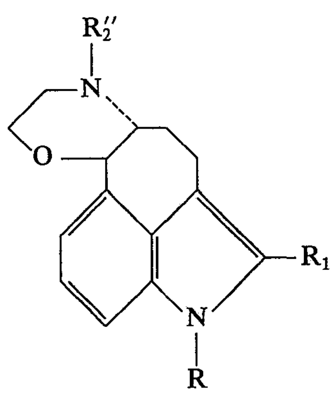

($I_B$)

dans laquelle R et $R_2''$ ont la signification déjà indiquée et $R_1'$ représente un atome de chlore ou de brome, correspondant à un produit de formule I, dans laquelle $R_1$ a la valeur de $R_1'$ et $R_2$ a la valeur de $R_2''$, produit de formule $I_B$ que l'on isole et, si désiré, salifie;

— soit, dans le cas où $R_2''$ représente un atome d'hydrogène, l'on traite le produit de formule $I_A$ par un halogènure de formule V:

Hal—$R_2'$ (V)

dans laquelle Hal représente un atome de chlore, de brome ou d'iode, et $R_2'$ a la signification de $R_2$

déjà indiquée à l'exception de l'hydrogène, pour obtenir un produit de formule $I_C$:

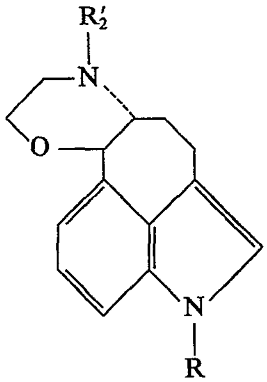

($I_C$)

dans laquelle R et $R_2'$ ont la signification déjà indiquée, correspondant à un produit de formule I, dans laquelle $R_2$ a les valeurs de $R_2'$ et $R_1$ représente un atome d'hydrogène, produit de formule $I_C$ que ou bien l'on isole et, si désiré, salifie, ou bien l'on traite à l'aide d'un agent d'halogénation, pour obtenir un produit de formule $I_D$:

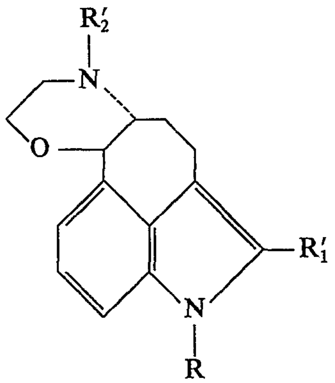

($I_D$)

dans laquelle R, $R_1'$ et $R_2'$ ont la signification déjà indiquée, correspondant à un produit de formule I, dans laquelle $R_1$ a les valeurs de $R_1'$ et $R_2$ a les valeurs de $R_2'$, produit de formule $I_D$ que l'on isole et, si désiré, salifie;

— soit, dans le cas où $R_2''$ représente un atome d'hydrogène, l'on traite le produit de formule $I_A$ par un agent de formylation pour obtenir un produit de formule VI:

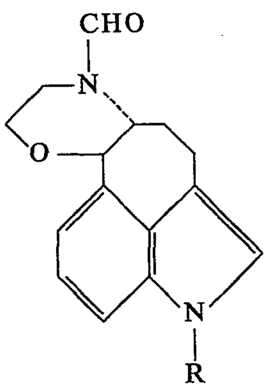

(VI)

dans laquelle R a la signification déjà indiquée, que l'on traite à l'aide d'un agent réducteur pour

obtenir un produit de formule $I_E$:

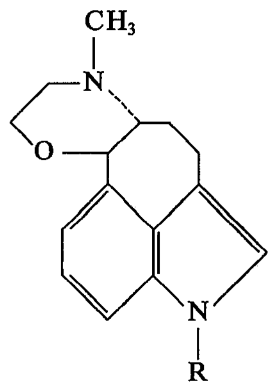

($I_E$)

dans laquelle R a la signification déjà indiquée, correspondant à un produit de formule I, dans laquelle $R_1$ représente un atome d'hydrogène et $R_2$ représente un radical méthyle, produit de formule $I_E$ que:
ou bien l'on isole et, si désiré, salifie;
ou bien l'on traite à l'aide d'un agent d'halogénation pour obtenir un produit de formule $I_F$:

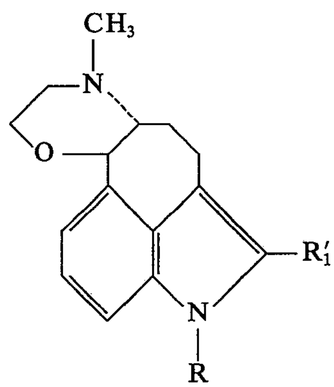

($I_F$)

dans laquelle R et $R_1'$ ont la signification déjà indiquée, correspondant à un produit de formule I, dans laquelle $R_1$ a les valeurs de $R_1'$ et $R_2$ représente un radical méthyle, produit de formule $I_F$ que l'on isole et, si désiré, salifie,

— soit, dans le cas où R représente un atome d'hydrogène et $R_2''$ a les valeurs telles que définies précédemment à l'exception d'hydrogène, l'on traite le produit de formule $I_A$ par un halogénure de formule

Hal—R'

dans laquelle Hal représente un atome de chlore, de brome ou d'iode et R' a la signification de R, à l'exception d'hydrogène, pour obtenir un produit de formule $I_G$:

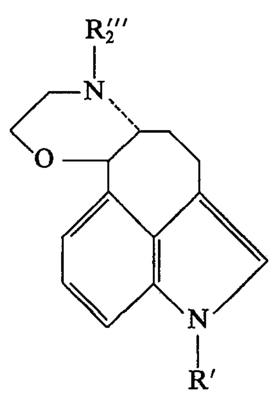

($I_G$)

dans laquelle R' a la signification déjà indiquée et $R_2'''$ a la signification de $R_2''$, à l'exception d'hydrogène, correspondant à un produit de formule I, dans laquelle R a les valeurs de R' et $R_2$ a les valeurs de $R_2'''$, produit de formule $I_G$ que ou bien l'on isole et, si désiré, salifie, ou bien l'on traite par un agent

d'halogénation, pour obtenir un produit de formule $I_H$:

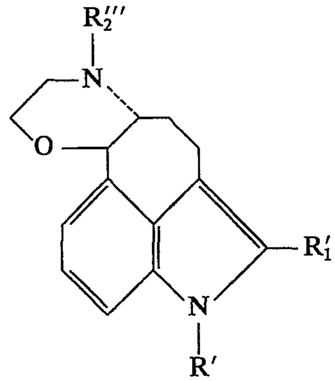

($I_H$)

dans laquelle R', $R_1$' et $R_2$''' ont la signification déjà indiquée, correspondant à un produit de formule I, dans laquelle R a les valeurs de R', $R_1$ celles de $R_1$' et $R_2$ celles de $R_2$''', produit de formule $I_H$ que l'on isole et, si désiré, salifie.

6. Procédé selon la revendication 5, caractérisé en ce que

— la cyclisation du produit de formule III est réalisée à l'aide d'hydrure de sodium;

— l'on réduit le produit de formule IV à l'aide d'hydrure d'aluminium-lithium;

— l'agent d'halogénation est, dans le cas de la bromation, le complexe bromé de la 2-pyrrolidone de formule:

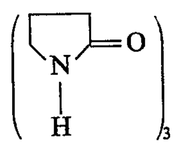

HBr $Br_2$

7. Médicaments, caractérisés en ce qu'ils sont constitués par les nouveaux dérivés du cycloheptindole tels que définis par la formule I de la revendication 1, ainsi que par leurs sels d'addition avec les acides pharmaceutiquement acceptables.

8. Médicaments, caractérisés en ce qu'ils sont constitués par les nouveaux dérivés du cycloheptindole, tels que définis à la revendication 2 ou 3, ainsi que par leurs sels d'addition avec les acides pharmaceutiquement acceptables.

9. Médicaments, caractérisés en ce qu'ils sont constitués par les dérivés du cycloheptindole, tels que définis à la revendication 4.

10. Compositions pharmaceutiques, caractérisées en ce qu'elles renfermant, à titre de principe actif, l'un au moins des médicaments, tels que définis à l'une quelconque des revendications 7, 8 ou 9.

**Revendications pour l'Etat contractant: AT**

1. Procédé de préparation des dérivés du cycloheptindole répondant à la formule générale I:

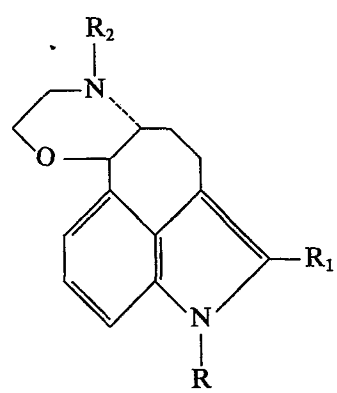

(I)

ainsi que de leurs sels d'addition avec les cides minéraux ou organiques, formule I dans laquelle R représente un atome d'hydrogène, un radical alcoyle renfermant de 1 à 5 atomes de carbone, ou un radical aralcoyle renfermant de 7 à 12 atomes de carbone, éventuellement substitué par des radicaux chloro, bromo, méthyl, éthyl, méthoxy, trifluorométhyl ou méthylthio, $R_1$ représente un atome d'hydrogène, de chlore ou de brome et $R_2$ représente un atome d'hydrogène, un radical alcoyle renfermant de 1 à 5 atomes de carbone, cycloalcoylalcoyle renfermant de 4 à 7 atomes de carbone, alcényle ou alcynyle renfermant de 3 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio, caractérisé en ce que l'on fait réagir un produit de formule II:

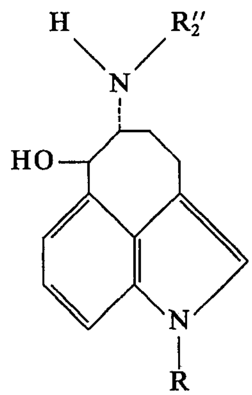

(II)

dans laquelle R a la signification déjà indiquée et $R_2''$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué, avec le chlorure de chloroacétyle, pour obtenir un produit de formule III:

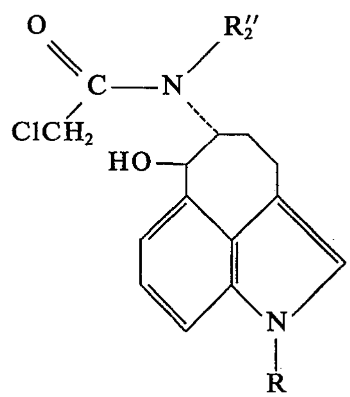

(III)

dans laquelle R et $R_2''$ ont la signification déjà indiquée, que l'on cyclise pour obtenir un produit de formule IV:

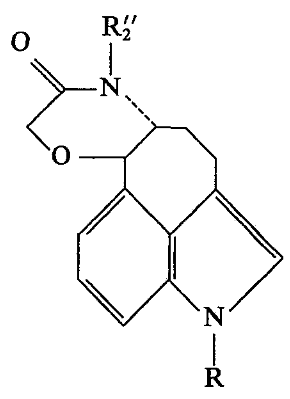

(IV)

dans laquelle R et $R_2''$ ont la signification déjà indiquée, que l'on réduit pour obtenir un produit de

formule $I_A$:

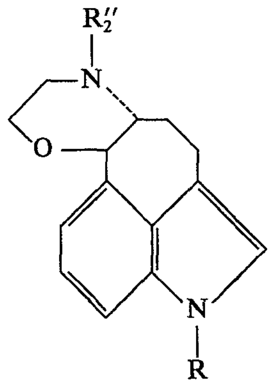

$(I_A)$

dans laquelle R et $R_2''$ ont la signification déjà indiquée, correspondant à un produit de formule I, dans laquelle $R_2$ a les valeurs de $R_2''$ et $R_1$ représente un atome d'hydrogène, produit de formule $I_A$, que:
— soit l'on isole et, si désiré, salifie,
— soit l'on traite le produit de formule $I_A$ à l'aide d'un agent d'halogénation, pour obtenir un produit de formule $I_B$:

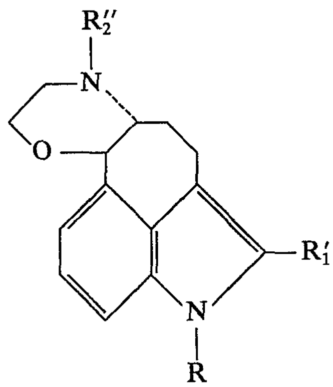

$(I_B)$

dans laquelle R et $R_2''$ ont la signification déjà indiquée et $R_1'$ représente un atome de chlore ou de brome, correspondant à un produit de formule I, dans laquelle $R_1$ a la valeur de $R_1'$ et $R_2$ a la valeur de $R_2''$, produit de formule $I_B$ que l'on isole et, si désiré, salifie;
— soit, dans le cas où $R_2''$ représente un atome d'hydrogène, l'on traite le produit de formule $I_A$ par un halogènure de formule V:

$$Hal—R_2' \quad (V)$$

dans laquelle Hal représente un atome de chlore, de brome ou d'iode, et $R_2'$ a la signification de $R_2$ déjà indiquée à l'exception de l'hydrogène, pour obtenir un produit de formule $I_C$:

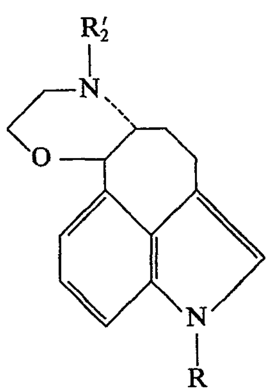

$(I_C)$

dans laquelle R et $R_2'$ ont la signification déjà indiquée, correspondant à un produit de formule I, dans laquelle $R_2$ a les valeurs de $R_2'$ et $R_1$ représente un atome d'hydrogène, produit de formule $I_C$ que ou bien l'on isole et, si désiré, salifie, ou bien l'on traite à l'aide d'un agent d'halogénation, pour obtenir un

produit de formule $I_D$:

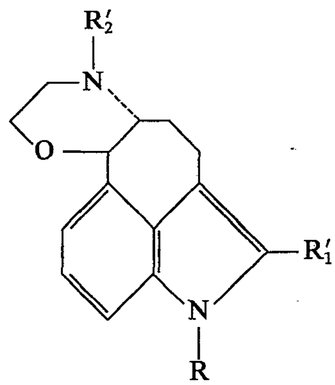

($I_D$)

dans laquelle R, $R_1'$ et $R_2'$ ont la signification déjà indiquée, correspondant à un produit de formule I, dans laquelle $R_1$ a les valeurs de $R_1'$ et $R_2$ a les valeurs de $R_2'$, produit de formule $I_D$ que l'on isole et, si désiré, salifie;

— soit, dans le cas où $R_2''$ représente un atome d'hydrogène, l'on traite le produit de formule $I_A$ par un agent de formylation pour obtenir un produit de formule VI:

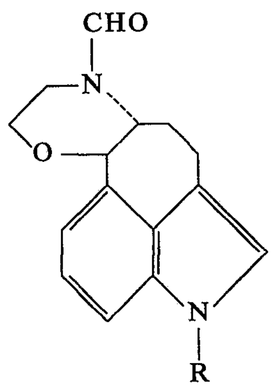

(VI)

dans laquelle R a la signification déjà indiquée, que l'on traite à l'aide d'un agent réducteur pour obtenir un produit de formule $I_E$:

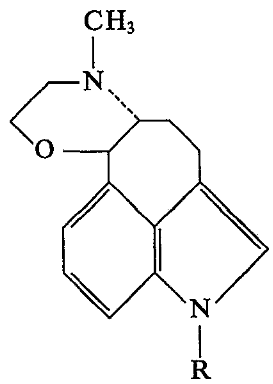

($I_E$)

dans laquelle R a la signification déjà indiquée, correspondant à un produit de formule I, dans laquelle $R_1$ représente un atome d'hydrogène et $R_2$ représente un radical méthyle, produit de formule $I_E$ que:
ou bien l'on isole et, si désiré, salifie;

ou bien l'on traite à l'aide d'un agent d'halogénation pour obtenir un produit de formule $I_F$:

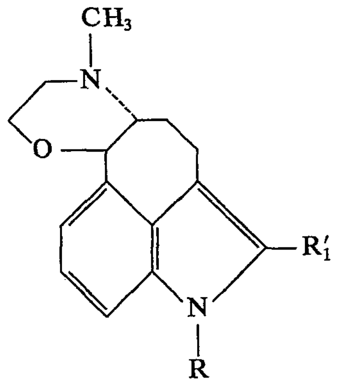

($I_F$)

dans laquelle R et $R_1'$ ont la signification déjà indiquée correspondant à ùn produit de formule I, dans laquelle $R_1$ a les valeurs de $R_1'$ et $R_2$ représente un radical méthyle, produit de formule $I_F$ que l'on isole et, si désiré, salifie,

— soit, dans le cas où R représente un atome d'hydrogène et $R_2''$ a les valeurs telles que définies précédemment à l'exception d'hydrogène, l'on traite le produit de formule $I_A$ par un halogénure de formule

Hal—R'

dans laquelle Hal représente un atome de chlore, de brome ou d'iode et R' a la signification de R, à l'exception d'hydrogène, pour obtenir un produit de formule $I_G$:

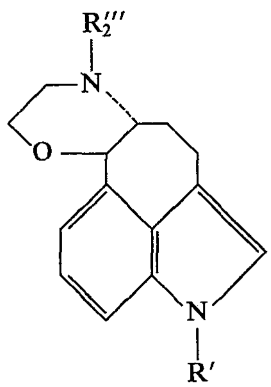

($I_G$)

dans laquelle R' a la signification déjà indiquée et $R_2''$ a la signification de $R_2''$, à l'exception d'hydrogène, correspondant à un produit de formule I, dans laquelle R a les valeurs de R' et $R_2$ a les valeurs de $R_2'''$, produit de formule $I_G$ que ou bien l'on isole et, si désiré, salifie, ou bien l'on traite par un agent d'halogénation, pour obtenir un produit de formule $I_H$:

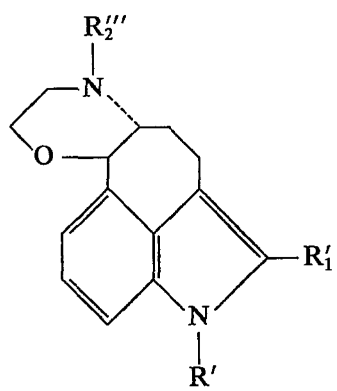

($I_H$)

dans laquelle R', $R_1'$ et $R_2'''$ ont la signification déjà indiquée, correspondant à un produit de formule I, dans laquelle R a les valeurs de R', $R_1$ celles de $R_1'$ et $R_2$ celles de $R_2'''$, produit de formule $I_H$ que l'on isole et, si désiré, salifie.

2. Procédé selon la rvendication 1, caractérisé en ce que

— la cyclisation du produit de formule III est réalisée à l'aide d'hydrure de sodium;
— l'on réduit le produit de formule IV à l'aide d'hydrure d'aluminium-lithium;
— l'agent d'halogénation est, dans le cas de la bromation, le complexe bromé de la 2-pyrrolidone de formule:

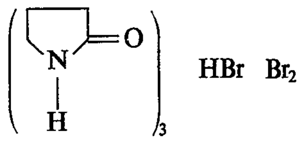

3. Procédé selon la revendication 1 ou 2, caractérisé en ce que l'on utilise au départ un produit de formule II dans laquelle R représente un atome d'hydrogène, un radical méthyle ou un radical benzyl et $R_2''$ représente un atome d'hydrogène ou un radical alcoyle renfermant de 1 à 5 atomes de carbone ou cycloalcoylalcoyle renfermant de 4 à 7 atomes de carbone.

4. Procédé selon la revendication 1 ou 2, caractérisé en ce que l'on utilise au départ un produit de formule II dans laquelle $R_2''$ représente un radical alcoyle renfermant de 1 à 5 atomes de carbone ou cycloalcoylalcoyle renfermant de 4 à 7 atomes de carbone et un produit de formule VI dans laquelle R' représente un radical méthyl ou un radical benzyl.

5. Procédé selon la revendication 1 ou 2, caractérisé en ce que l'on utilise au départ un produit de formule II dans laquelle R représente un atome d'hydrogène et $R_2''$ représente un atome d'hydrogène ou un radical alcoyle renfermant de 1 à 5 atomes de carbone.

**Patentansprüche für die Vertragsstaaten BE, CH, DE, FR, GB, IT, LI, LU, NL, SE**

1. Neue Cycloheptindolderivate sowie deren Additionssalze mit Mineral- oder oganischen Säuren, daduch gekennzeichnet, daß sie der allgemeinen Formel I

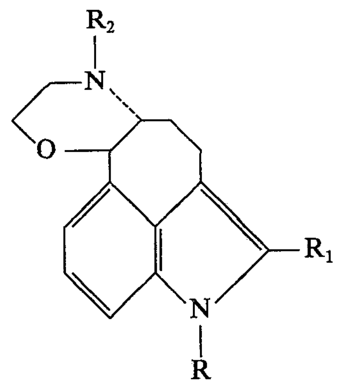

(I)

entsprechen, worin R ein Wasserstoffatom, einen Alkylrest mit 1 bis 5 Kohlenstoffatomen oder einen Aralkylrest mit 7 bis 12 Kohlensstofffatomen, gegebenenfalls substituiert duch Chlor-, Brom-, Methyl-, Ethyl-, Methoxy-, Trifluormethyl- oder Methylthioreste, bedeutet; $R_1$ ein Wasserstoff-, Chlor- oder Bromatom darstellt; und $R_2$ ein Wasserstoffatom, einen Alkylrest mit 1 bis 5 Kohlenstoffatomen, einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet.

2. Neue Cycloheptindolderivate gemäß Anspruch 1 sowie deren Additionssalze mit Mineral- oder organischen Säuren, dadurch gekennzeichnet, daß R ein Wasserstoffatom, einen Methylrest oder einen Benzylrest bedeutet, $R_1$ wie in Anspruch 1 definiert ist und $R_2$ ein Wasserstoffatom oder einen Alkylrest mit 1 bis 5 Kohlenstoffatomen oder einen Cycloalkalkylrest mit 4 bis 7 Kohlenstoffatomen bedeutet.

3. Neue Cycloheptindolderivate gemäß Anspruch 1 sowie deren Addditionssalze mit Mineral- oder organischen Säuren, dadurch gekennzeichnet, daß R ein Wasserstoffatom bedeutet, $R_1$ ein Wasserstoff- oder Bromatom darstellt und $R_2$ ein Wasserstoffatom oder einen Alkylrest mit 1 bis 5 Kohlenstoffatomen bedeutet.

4. Derivat der Formel (I) gemäß Anspruch 1 mit der folgenden Bezeichnung:
(7a-RS-trans)-8-Methyl-4,6,7,7a,8,9,10,11a-octahydro[1.4]oxazino[2'.3'-4.5]-cyclohept[1.2.3-c.d]indol
sowie dessen Additionssalze mit Mineral- oder organischen Säuren.

5. Verfahren zur Herstellung der Derivate der Formel I gemäß Anspruch 1 sowie von deren Additionssalzen mit Mineral- oder organischen Säuren, dadurch gekennzeichnet, daß man ein Produkt der

Formel II

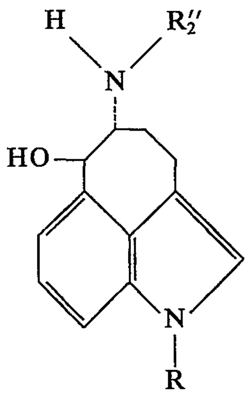

(II)

worin R die angegebene Bedeutung besitzt und $R_2''$ ein Wassersstoffatom, einen Alkylrest mit 1 bis 5 Kohlenstoffatomen, einen Cycloalkalkylrest mit 4 bis 7 Kohlenstoffatomen oder einen Aralkylrest mit 7 bis 12 Kohlenstoffatomen, gegebenenfalls substituiert, bedeutet, mit Chloracetylchlorid umsetzt, um ein Produkt der Formel III

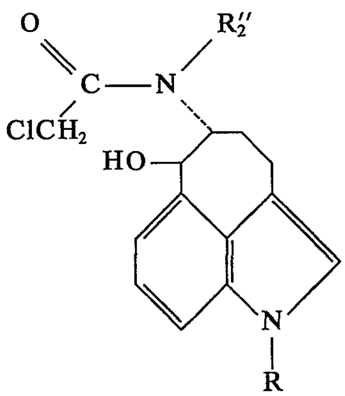

(III)

zu erhalten, worin R und $R_2''$ die angegebene Bedeutung besitzen, das man cyclisiert, um ein Produkt der Formel IV

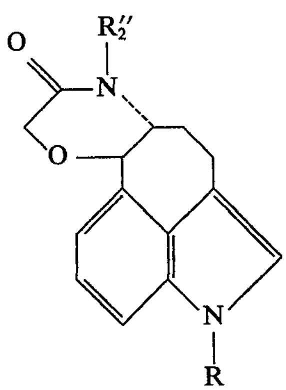

(IV)

zu erhalten, worin R und $R_2''$ die angegebene Bedeutung besitzen, das man reduziert, um ein Produkt

der Formel $I_A$

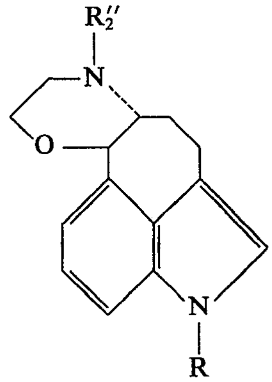

($I_A$)

zu erhalten, worin R und $R_2''$ die angegebene Bedeutung besitzen, entsprechend einem Produkt der Formel I, worin $R_2$ die Bedeutungen von $R_2''$ besitzt und $R_1$ ein Wasserstoffatom bedeutet, welches Produkt der Formel $I_A$:

man entweder isoliert und gewünschtenfalls in ein Salz überführt,

oder man behandelt das Produkt der Formel $I_A$ mit einem Halogenierungsmittel, um ein Produkt der Formel $I_B$

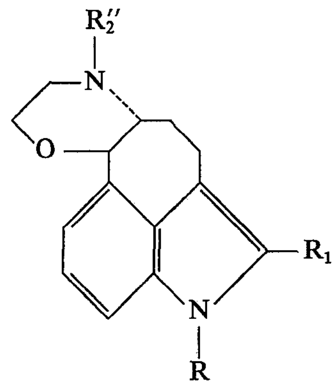

($I_B$)

zu erhalten, worin R und $R_2''$ die angegebene Bedeutung besitzenn und $R_1'$ ein Chlor- oder Bromatom bedeutet, entsprechend einem Produkt der Formel I, worin $R_1$ die Bedeutung von $R_1'$ besitzt und $R_2$ die Bedeutung von $R_2''$ hat, welches Produkt der Formel ($I_B$) man isoliert und gewünschtenfalls in ein Salz überführt,

oder, wenn $R_2''$ ein Wasserstoffatom bedeutet, man das Produkt der Formel $I_A$ mit einem Halogenid der Formel V

$$Hal-R_2' \qquad (V)$$

worin Hal ein Chlor-, Brom- oder Jodatom bedeutet und $R_2'$ die für $R_2$ angegebene Bedeutung mit Ausnahme von Wasserstoff besitzt, behandelt, um ein Produkt der Formel $I_C$

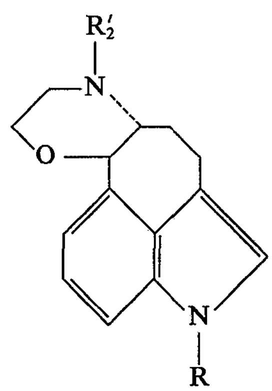

($I_C$)

zu erhalten, worin R und $R_2'$ die angegebene Bedeutung besitzen, entsprechend einem Produkt der Formel I, worin $R_2$ die Bedeutungen von $R_2'$ besitzt und $R_1$ ein Wasserstoffatom bedeutet, welches Produkt der Formel $I_C$ man entweder isoliert oder gewünschtenfalls in ein Salz überführt oder mit

einem Halogenierungsmittel behandelt, um ein Produkt der Formel $I_D$

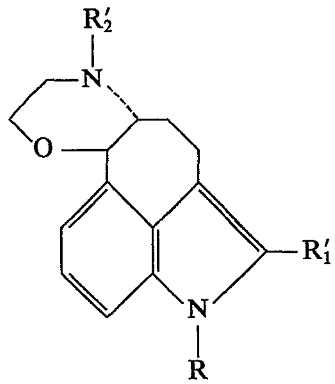

($I_D$)

zu erhalten, worin R, $R_1'$ und $R_2'$ die angegebene Bedeutung besitzen, entsprechend einem Produkt der Formel I, worin $R_1$ die Bedeutungen von $R_1'$ besitzt und $R_2$ die Bedeutungen von $R_2'$ besitzt, welches Produkt der Formel $I_D$ man isoliert und gewünschtenfalls in ein Salz überführt,

oder, wenn $R_2''$ ein Wasserstoffatom bedeutet, man das Produkt der Formel $I_A$ mit einem Formylierungsmittel behandelt, um ein Produkt der Formel VI

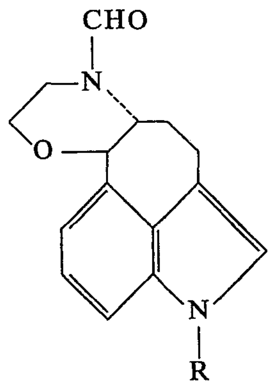

(VI)

zu erhalten, worin R die angegebene Bedeutung hat, das man mit einem Reduktionsmittel behandelt, um ein Produkt der Formel $I_E$

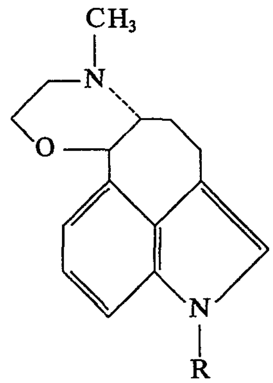

($I_E$)

zu erhalten, worin R die angegebene Bedeutung besitzt, entsprechend einem Produkt der Formel I, worin $R_1$ ein Wasserstoffatom bedeutet und $R_2$ einen Methylrest darstellt, welches Produkt der Formel $I_E$:

man entweder isoliert und gewünschtenfalls in ein Salz überführt,

oder mit einem Halogenierungsmittel behandelt, um ein Produkt der Formel $I_F$

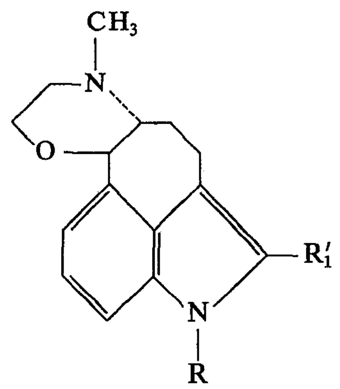

($I_F$)

zu erhalten, worin R und $R_1'$ die angegebene Bedeutung besitzen, entsprechend einem Produkt der Formel I, worin $R_1$ die Bedeutungen von $R_1'$ besitzt und $R_2$ einen Methylrest bedeutet, welches Produkt der Formel $I_F$ man isoliert und gewünschtenfalls in ein Salz überführt,

oder, wenn R ein Wasserstoffatom bedeutet und $R_2''$ die vorstehend angegebenen Bedeutungen mit Ausnahme von Wasserstoff besitzt, man das Produkt der Formel $I_A$ mit einem Halogenid der Formel

Hal—R′

worin Hal ein Chlor-, Brom oder Jodatom bedeutet und R′ die Bedeutung von R mit Ausnahme von Wasserstoff besitzt, behandelt, um ein Produkt der Formel $I_G$

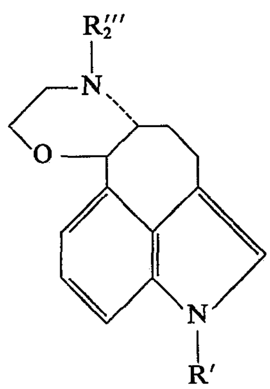

($I_G$)

zu erhalten, worin R′ die angegebene Bedeutung besitzt und $R_2'''$ die Bedeutung von $R_2''$ mit Ausnahme von Wasserstoff besitzt, entsprechend einem Produkt der Formel I, worin R die Bedeutungen von R′ besitzt und $R_2$ die Bedeutungen von $R_2'''$ besitzt, welches Produkt der Formel $I_G$ man entweder isoliert und gewünschtenfalls in ein Salz überführt oder mit einem Halogenierungsmittel behandelt, um ein Produkt der Formel $I_H$

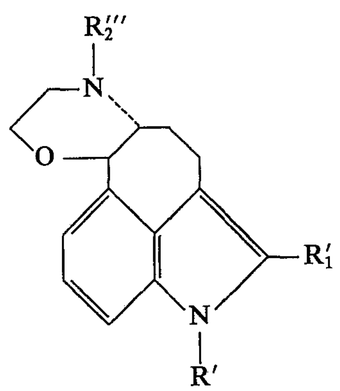

($I_H$)

zu erhalten, worin R′, $R_1'$ und $R_2''$ die angegebene Bedeutung besitzen, entsprechend einem Produkt der Formel I, worin R die Bedeutungen von R′, $R_1$ diejenigen von $R_1'$ und $R_2$ diejenigen von $R_2'''$ besitzt, welches Produkt der Formel $I_H$ man isoliert und gewünschtenfalls in ein Salz überführt.

6. Verfahren gemäß Anspruch 5, dadurch gekennzeichnet, daß

die Cyclisierung des Produktes der Formel III mit Hilfe von Natriumhydrid durchgeführt wird,
man das Produkt der Formel IV mit Aluminiumlithiumhydrid reduziert,
das Halogenierungsmittel im Falle der Bromierung der Bromkomplex von 2-Pyrrolidon der Formel

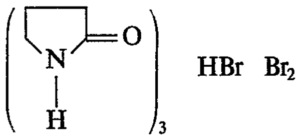

ist.

7. Arzneimittel, dadurch gekennzeichnet, daß sie aus den neuen Cycloheptindolderivaten der Formel I gemäß Anspruch 1 oder aus deren Additionssalzen mit pharmazeutisch verträglichen Säuren bestehen.

8. Arzneimittel, dadurch gekennzeichnet, daß sie aus den neuen Cycloheptindolderivaten gemäß Anspruch 2 oder 3 oder aus deren Additionssalzen mit pharmazeutisch verträglichen Säuren bestehen.

9. Arzneimittel, dadurch gekennzeichnet, daß sie aus den Cycloheptindolderivaten gemäß Anspruch 4 bestehen.

10. Pharmazeutische Zusammensetzungen, dadurch gekennzeichnet, daß sie als Wirkstoff zumindest eines der Arzneimittel gemäß einem der Ansprüche 7, 8 oder 9 enthalten.

**Patentansprüche für den Vertragsstaat: AT**

1. Verfahren zur Herstellung von Cycloheptindolderivaten der allgemeinen Formel I

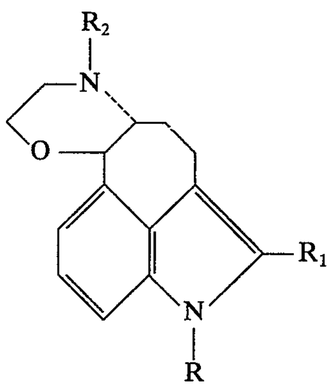

(I)

sowie von deren Additionssalzen mit Mineral- oder organischen Säuren, wobei in der Formel I R ein Wasserstoffatom, einen Alkylrest mit 1 bis 5 Kohlenstoffatomen oder einen Aralkylrest mit 7 bis 12 Kohlenstoffatomen, gegebenenfalls substituiert durch Chlor-, Brom-, Methyl-, Ethyl-, Methoxy-, Trifluormethyl- oder Methylthioreste, bedeutet, $R_1$ ein Wasserstoff-, Chlor- oder Bromatom darstellt und $R_2$ ein Wasserstoffatom, einen Alkylrest mit 1 bis 5 Kohlenstoffatomen, einen Cycloalkalkylrest mit 4 bis 7 Kohlenstoffatomen, einen Alkenyl- oder Alkinylrest mit 3 bis 7 Kohlenstoffatomen oder einen Aralkylrest mit 7 bis 12 Kohlenstoffatomen, gegebenenfalls substituiert durch Brom-, Chlor-, Methyl-, Ethyl-, Methoxy-, Trifluormethyl- oder Methylthioreste, bedeutet, dadurch gekennzeichnet, daß man ein Produkt der Formel II

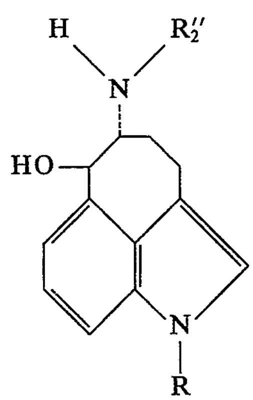

(II)

worin R die angegebene Bedeutung besitzt und $R_2''$ ein Wasserstoffatom, einen Alkylrest mit 1 bis 5 Kohlenstoffatomen, einen Cycloalkalkylrest mit 4 bis 7 Kohlenstoffatomen oder einen Aralkylrest mit 7 bis 12 Kohlenstoffatomen, gegebenenfalls substituiert, bedeutet, mit Chloracetylchlorid umsetzt, um

ein Produkt der Formel III

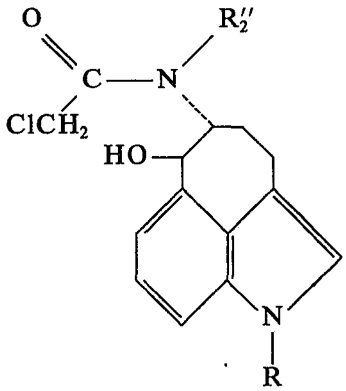

(III)

zu erhalten, worin R und $R_2''$ die angegebene Bedeutung besitzen, das man cyclisiert, um ein Produkt der Formel IV

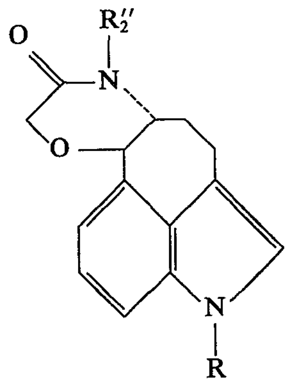

(IV)

zu erhalten, worin R und $R_2''$ die angegebene Bedeutung besitzen, welches man reduziert, um ein Produkt der Formel $I_A$

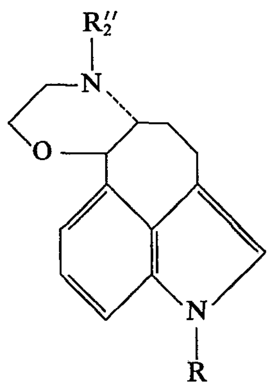

($I_A$)

zu erhalten, worin R und $R_2''$ die angegebene Bedeutung besitzen, entsprechend einem Produkt der Formel I, worin $R_2$ die Bedeutungen von $R_2''$ besitzt und $R_1$ ein Wasserstoffatom bedeutet, welches Produkt der Formel $I_A$:

man entweder isoliert und gewünschtenfalls in ein Salz überführt,

oder man behandelt das Produkt der Formel $I_A$ mit einem Halogenierungsmittel, um ein Produkt der

Formel $I_B$

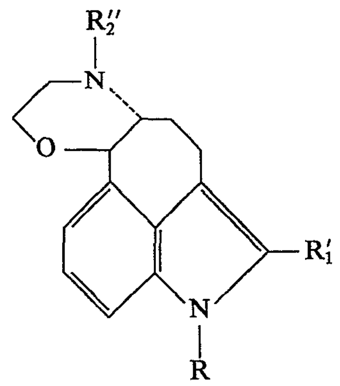

($I_B$)

zu erhalten, worin R und $R_2''$ die angegebene Bedeutung besitzen und $R_1$ ein Chlor- oder Bromatom bedeutet, entsprechend einem Produkt der Formel I, worin $R_1$ die Bedeutung von $R_1'$ besitzt und $R_2$ die Bedeutung von $R_2''$ hat, welches Produkt der Formel $I_B$ man isoliert und gewünschtenfalls in ein Salz überführt,

oder, wenn $R_2''$ ein Wasserstoffatom bedeutet, man das Produkt der Formel $I_A$ mit einem Halogenid der Formel V

$$Hal—R_2' \qquad (V)$$

behandelt, worin Hal ein Chlor-, Brom oder Jodatom bedeutet und $R_2'$ die Bedeutung von $R_2$ mit Ausnahme von Wasserstoff besitzt, um ein Produkt der Formel $I_C$

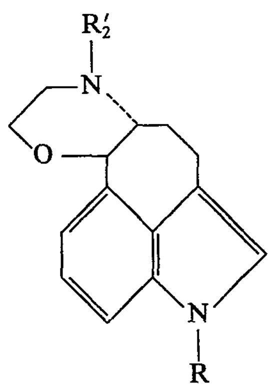

($I_C$)

zu erhalten, worin R und $R_2'$ die angegebene Bedeutung besitzen, entsprechend einem Produkt der Formel I, worin $R_2$ die Bedeutungen von $R_2'$ besitzt und $R_1$ ein Wasserstoffatom bedeutet, welches Produkt der Formel $I_C$ man entweder isoliert und gewünschtenfalls in ein Salz überführt oder mit einem Halogenierungsmittel behandelt, um ein Produkt der Formel $I_D$

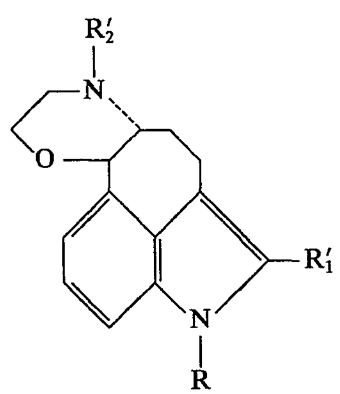

($I_D$)

zu erhalten, worin R, $R_1'$ und $R_2'$ die angegebene Bedeutung besitzen, entsprechend einem Produkt der Formel I, worin $R_1$ die Bedeutungen von $R_1'$ besitzt und $R_2$ die Bedeutungen von $R_2'$ hat, welches Produkt der Formel $I_D$ man isoliert und gewünschtenfalls in ein Salz überführt;

oder, wenn $R_2''$ ein Wasserstoffatom bedeutet, man das Produkt der Formel $I_A$ mit einem Formylierungsmittel behandelt, um ein Produkt der Formel VI

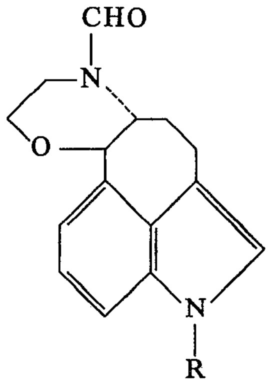

(VI)

zu erhalten, worin R die angegebene Bedeutung hat, das man mit einem Reduktionsmittel behandelt, um ein Produkt der Formel $I_E$

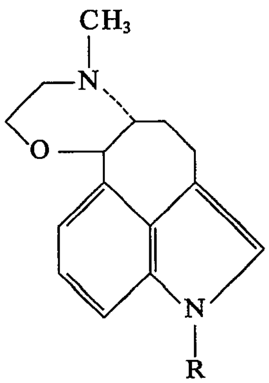

($I_E$)

zu erhalten, worin R die angegebene Bedeutung besitzt, entsprechend einem Produkt der Formel I, worin $R_1$ ein Wasserstoffatom bedeutet und $R_2$ einen Methylrest darstellt, welches Produkt der Formel $I_E$:

man entweder isoliert und gewünschtenfalls in ein Salz überführt,

oder mit einem Halogenierungsmittel behandelt, um ein Produkt der Formel $I_F$

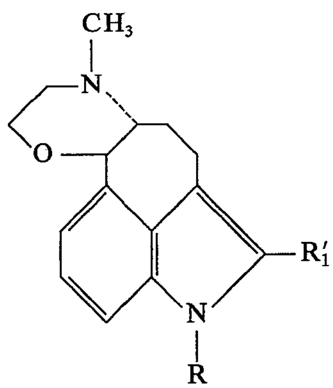

($I_F$)

zu erhalten, worin R und $R_1'$ die angegebene Bedeutung besitzen, entsprechend einem Produkt der Formel I, worin $R_1$ die Bedeutungen von $R_1'$ besitzt und $R_2$ einen Methylrest bedeutet, welches Produkt der Formel $I_F$ man isoliert und gewünschtenfalls in ein Salz überführt,

oder, wenn R ein Wasserstoffatom bedeutet und $R_2''$ die vorstehend angegebenen Bedeutungen mit Ausnahme von Wasserstoff besitzt, man das Produkt der Formel $I_A$ mit einem Halogenid der Formel

Hal—R'

worin Hal ein Chlor-, Brom- oder Jodatom bedeutet und R' die Bedeutung von R mit Ausnahme von

Wasserstoff besitzt, behandelt, um ein Produkt der Formel $I_G$

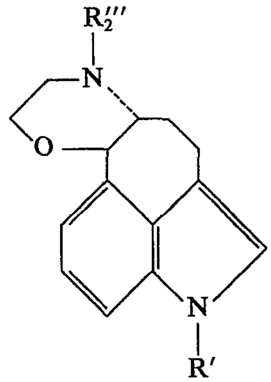

($I_G$)

zu erhalten, worin R' die angegebene Bedeutung besitzt und $R_2'''$ die Bedeutung von $R_2''$ mit Ausnahme von Wasserstoff besitzt, entsprechend einem Produkt der Formel I, worin R die Bedeutung von R' und $R_2$ die Bedeutungen von $R_2'''$ besitzt, welches Produkt der Formel $I_G$ man entweder isoliert und gewünschtenfalls in ein Salz überführt oder man mit einem Halogenierungsmittel behandelt, um ein Produkt der Formel $I_H$

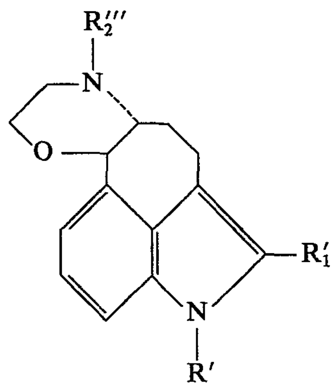

($I_H$)

zu erhalten, worin R', $R_1'$ und $R_2'''$ die angegebene Bedeutung besitzen, entsprechend einem Produkt der Formel I, worin R die Bedeutungen von R', $R_1$ diejenigen von $R_1'$ und $R_2$ diejenigen von $R_2'''$ besitzt, welches Produkt der Formel $I_H$ man isoliert und gewünschtenfalls in ein Salz überführt.

2. Verfahren gemäß Anspruch 1, dadurch gekennzeichnet, daß
die Cyclisierung des Produktes der Formel III mit Natriumhydrid durchgeführt wird,
man das Produkt der Formel IV mit Aluminiumlithiumhydrid reduziert,
das Halogenierungsmittel im Falle der Bromierung der Bromkomplex von 2-Pyrrolidon der Formel

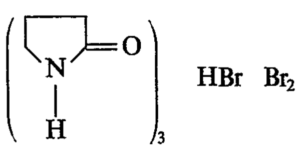

ist.

3. Verfahren gemäß Anspruch 1 oder 2, dadurch gekennzeichnet, daß man als Ausgangssubstanz ein Produkt der Formel II einsetzt, worin R ein Wasserstoffatom, einen Methylrest oder einen Benzylrest bedeutet und $R_2''$ ein Wasserstoffatom oder einen Alkylrest mit 1 bis 5 Kohlenstoffatomen oder einen Cycloalkalkylrest mit 4 bis 7 Kohlenstoffatomen bedeutet.

4. Verfahren gemäß Anspruch 1 oder 2, dadurch gekennzeichnet, daß man als Ausgangssubstanz ein Produkt der Formel II, worin $R_2''$ einen Alkylrest mit 1 bis 5 Kohlenstoffatomen oder einen Cycloalkalkylrest mit 4 bis 7 Kohlenstoffatomen bedeutet, und ein Produkt der Formel VI, worin R' einen Methylrest oder einen Benzylrest bedeutet, verwendet.

5. Verfahren gemäß Anspruch 1 oder 2, dadurch gekennzeichnet, daß man als Ausgangsstoff ein Produkt der Formel II verwendet, worin R ein Wasserstoffatom darstellt und $R_2''$ ein Wasserstoffatom oder einen Alkylrest mit 1 bis 5 Kohlenstoffatomen darstellt.

**Claims for the contracting States: BE, CH, DE, FR, GB, IT, LI, LU, NL, SE**

1. New derivatives of cycloheptindole, as well as their salts of addition with mineral or organic acids, characterized in that they correspond to the general formula (I):

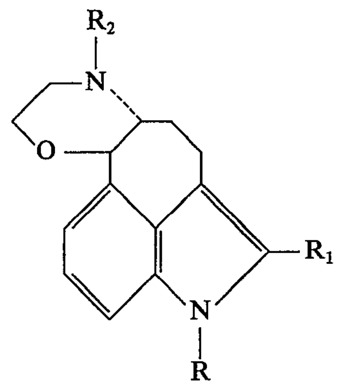

(I)

in which R represents a hydrogen atom, an alkyl radical containing 1—5 carbon atoms, or an aralkyl radical containing 7—12 carbon atoms, possibly sustituted by a chloro, bromo, methyl, ethyl, methoxy, trifluoromethyl or methylthio radical, $R_1$ represents a hydrogen, chlorine or bromine atom, and $R_2$ represents a hydrogen atom, an alkyl radical containing 1—5 carbon atoms, a cycloalkylalkyl radical containing 4—7 carbon atoms, an alkenyl radical or an alkynyl radical containing 3—7 carbon atoms, or an aralkyl radical containing 7—12 carbon atoms, possibly substituted by a chloro, bromo, methyl, ethyl, methoxy, trifluoromethyl or methylthio radical.

2. New derivatives of cycloheptindole according to Claim 1, as well as their salts of addition with mineral or organic acids, characterized in that R represents a hydrogen atom, a methyl radical or a benzyl radical, $R_1$ is as defined in Claim 1 and $R_2$ represents a hydrogen atom or an alkyl radical containing 1—5 carbon atoms or a cyclo-alkylalkyl radical containing 4—7 carbon atoms.

3. New derivatives of cycloheptindole, according to Claim 1, as well as their salts of addition with mineral or organic acids, characterized in that R represents a hydrogen atom, $R_1$ represents a hydrogen atom or a bromine atom, and $R_2$ represents a hydrogen atom or an alkyl radical containing 1—5 carbon atoms.

4. The derivative with the formula (I) as defined in Claim 1, the name of which follows:

— (7a RS trans) 8-methyl-4,6,7,7a,8,9,10,11a-octahydro-[-1,4-]-oxazino-[-2',3'-4,5-]-cyclohept-[-1,2,3-c,d]-indole,

as well as its salts of addition with mineral or organic acids.

5. Preparation process for the derivatives with the formula (I), as defined in Claim 1, as well as their salts of addition with mineral or organic acids, characterized in that a product with the formula (II):

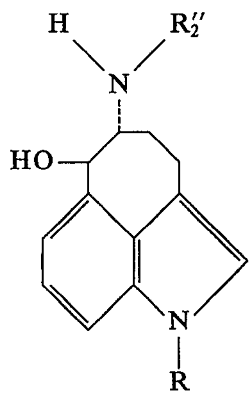

(II)

in which R has the significance already indicated and $R_2''$ represents a hydrogen atom, an alkyl radical containing 1—5 carbon atoms, a cycloalkylalkyl radical containing 4—7 carbon atoms, or an aralkyl radical containing 7—12 carbon atoms, possibly substituted, is made to react with chloroacetyl chloride, so as to obtain a product with the formula (III):

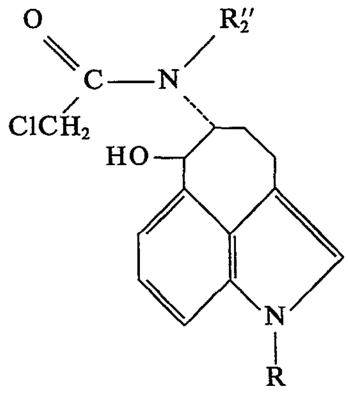

(III)

in which R and $R_2''$ have the significance already indicated, which is cyclized so as to obtain a product with the formula (IV):

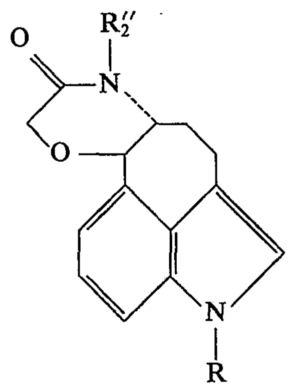

(IV)

in which R and $R_2''$ have the significance already indicated, which is reduced so as to obtain a product with the formula ($I_A$):

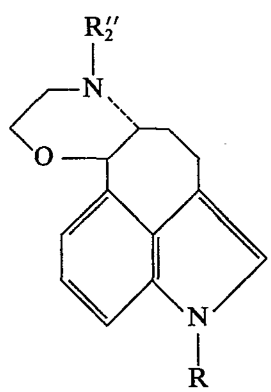

($I_A$)

in which R and $R_2''$ have the significance already indicated, corresponding to a product with the formula (I), in which $R_2$ has the values of $R_2''$ and $R_1$ represents a hydrogen atom, which product with the formula ($I_A$):

— either is isolated and, if desired, salified,

— or, the product with the formula ($I_A$) is treated by means of a halogenation agent, so as to obtain a

product with the formula ($I_B$):

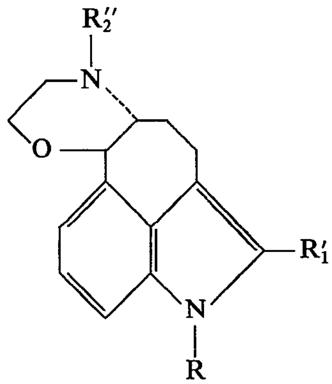

($I_B$)

in which R and $R_2''$ have the significance already indicated and $R_1'$ represents a chlorine or bromine atom, corresponding to a product with the formula (I), in which $R_1$ has the value of $R_1'$ and $R_2$ has the value of $R_2''$, which product with the formula ($I_B$) is isolated and, if desired, salified;
— or, when $R_2''$ represents a hydrogen atom, the product with the formula ($I_A$) is treated with a halogenide with the formula (V):

$$Hal-R_2' \quad (V)$$

in which Hal represents a chlorine, bromine or iodine atom and $R_2'$ has the significance of $R_2$ already indicated with the exception of hydrogen, so as to obtain a product with the formula ($I_C$)

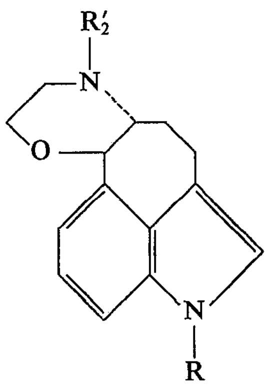

($I_C$)

in which R and $R_2'$ have the significance already indicated, corresponding to a product with the formula (I), in which $R_2$ has the values of $R_2'$ and $R_1$ represents a hydrogen atom, which product with the formula ($I_C$) either is isolated and, if desired, salified, or is treated by means of a halogenation agent, so as to obtain a product with the formula ($I_D$):

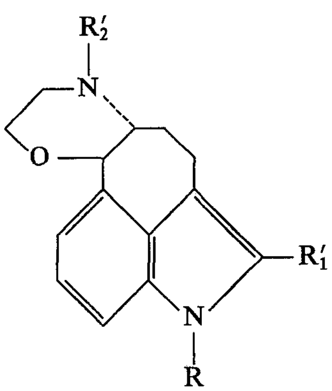

($I_D$)

in which R, $R_1'$ and $R_2'$ have the significance already indicated, corresponding to a product with the formula (I), in which $R_1$ has the values of $R_1'$ and $R_2$ has the values of $R_2'$, which product with the formula ($I_D$) is isolated and, if desired, salified;
— or, when $R_2''$ represents a hydrogen atom, the product with the formula ($I_A$) is treated with a

formylation agent so as to obtain a product with the formula (VI):

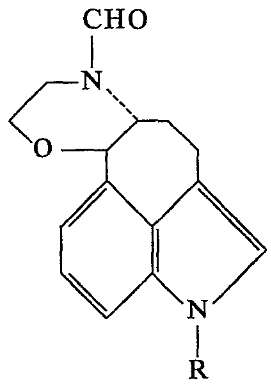

(VI)

in which R has the significance already indicated, which is treated by means of a reducing agent so as to obtain a product with the formula ($I_E$):

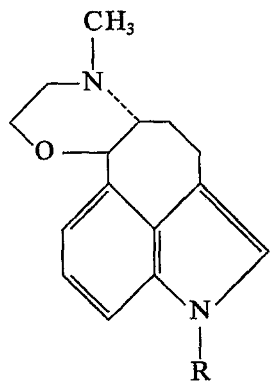

($I_E$)

in which R has the significance already indicated, corresponding to a product with the formula (I), in which $R_1$ represents a hydrogen atom and $R_2$ represents a methyl radical, which product with the formula ($I_E$)

either is isolated and, if desired, salified;

or is treated by means of a halogenation agent so as to obtain a product with the formula ($I_F$):

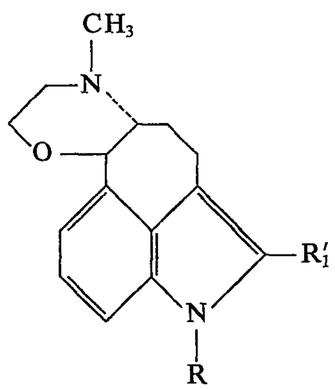

($I_F$)

in which R and $R_1'$ have the significance already indicated, corresponding to a product with the formula (I), in which $R_1$ has the values of $R_1'$ and $R_2$ represents a methyl radical, which product with the formula ($I_F$) is isolated and, if desired, salified,

— or, when R represents a hydrogen atom and $R_2''$ has the values as defined previously with the exception of hydrogen, the product with the formula ($I_A$) is treated by a halogenide with the formula

Hal—R′

in which Hal represents a chlorine, bromine or iodine atom and R′ has the significance of R, with the

exception of hydrogen, so as to obtain a product with the formula ($I_G$):

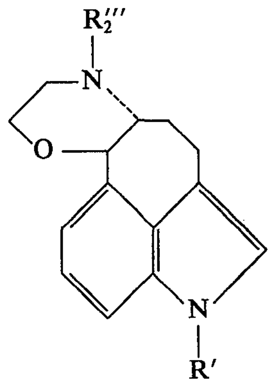

($I_G$)

in which R′ has the significance already indicated and $R_2'''$ has the significance of $R_2''$, with the exception of hydrogen, corresponding to a product with the formula (I), in which R has the values of R′ and $R_2$ has the values of $R_2'''$, which product with the formula ($I_G$) either is isolated and, if desired, salified, or is treated by a halogenation agent so as to obtain a product with the formula ($I_H$):

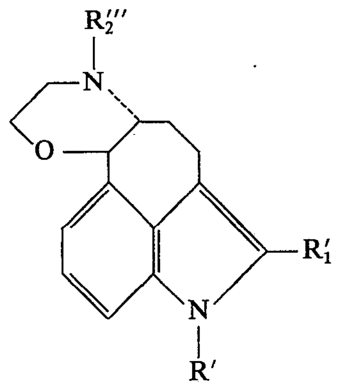

($I_H$)

in which R′, $R_1'$ and $R_2'''$ have the significance already indicated, corresponding to a product with the formula (I), in which R has the values of R′, $R_1$ has those of $R_1'$ and $R_2$ has those of $R_2'''$, which product with the formula ($I_H$) is isolated and, if desired, salified.

6. Process according to Claim 5, characterized in that

— cyclization of the product with the formula (III) is carried out by means of sodium hydride;

— the product with the formula (IV) is reduced by means of aluminium-lithium hydride;

— the halogenation agent is, in the case of bromation, the bromated complex of 2-pyrrolidone with the formula:

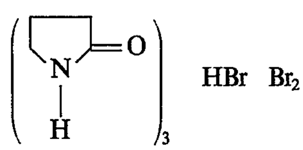

7. Medicaments, characterized in that they are constituted by new derivatives of cycloheptindole as defined by formula (I) of Claim 1, as well as by their salts of addition with pharmaceutically acceptable acids.

8. Medicaments, characterized in that they are constituted by new derivatives of cycloheptindole, as defined in Claim 2 or 3, as well as by their salts of addition with pharmaceutically acceptable acids.

9. Medicaments, characterized in that they are constituted by derivatives of cycloheptindole, as defined in Claim 4.

10. Pharmaceutical compositions, characterized in that they contain, as active principle, one at least of the medicaments as defined in any one of the Claims 7, 8 or 9.

**Claims for the contracting State: AT**

1. Preparation process for the derivatives of cycloheptindole corresponding to the general formula (I):

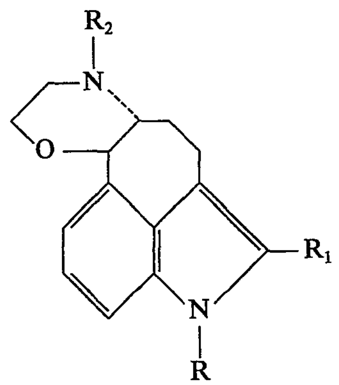

(I)

as well as their salts of addition with mineral or organic acids, in which formula (I) R represents a hydrogen atom, an alkyl radical containing 1—5 carbon atoms, or an aralkyl radical containing 7—12 carbon atoms, possibly substituted by a chloro, bromo, methyl, ethyl, methoxy, trifluoromethyl or methylthio radical, $R_1$ represents a hydrogen, chlorine or bromine atom and $R_2$ represents a hydrogen atom, an alkyl radical containing 1—5 carbon atoms, a cycloalkylalkyl radical containing 4—7 carbon atoms, an alkenyl or alkynyl radical containing 3—7 carbon atoms, or an aralkyl radical containing 7—12 carbon atoms, possibly substituted by a chloro, bromo, methyl, ethyl, methoxy, trifluoromethyl or methylthio radical, characterized in that a product with the formula (II):

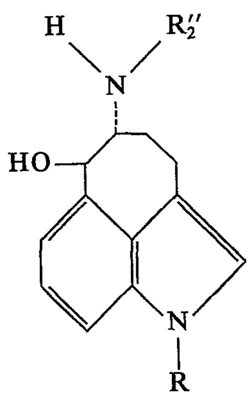

(II)

in which R has the significance already indicated and $R_2''$ represents a hydrogen atom, an alkyl radical containing 1—5 carbon atoms, a cycloalkylalkyl radical containing 4—7 carbon atoms or an aralkyl radical containing 7—12 carbon atoms, possibly substituted, is made to react with chloroacetyl chloride, so as to obtain a product with the formula (III):

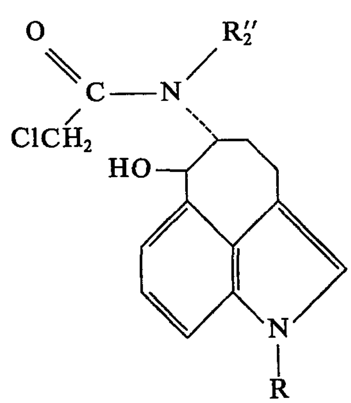

(III)

in which R and $R_2''$ have the significance already indicated, which is cyclized so as to obtain a product

with the formula (IV):

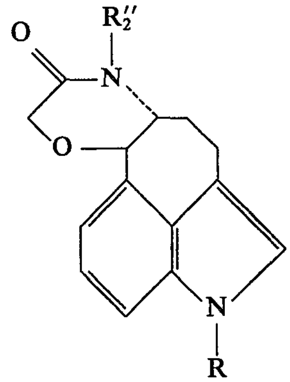

(IV)

in which R and $R_2''$ have the significance already indicated, which is reduced so as to obtain a product with the formula ($I_A$):

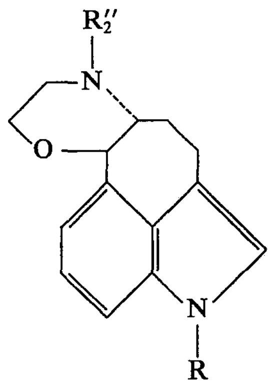

($I_A$)

in which R and $R_2''$ have the significance already indicated, corresponding to a product with the formula (I), in which $R_2$ has the values of $R_2''$ and $R_1$ represents a hydrogen atom, which product with the formula ($I_A$):
— either is isolated and, if desired, salified,
— or the product with the formula ($I_A$) is treated by means of a halogenation agent, so as to obtain a product with the formula ($I_B$):

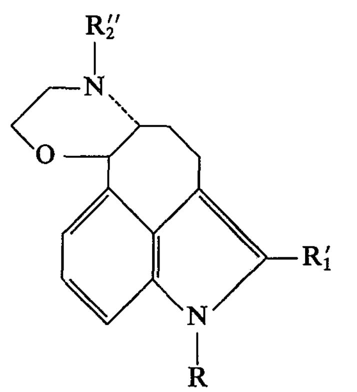

($I_B$)

in which R and $R_2''$ have the significance already indicated and $R_1'$ represents a chlorine or bromine atom, corresponding to a product with the formula (I), in which $R_1$ has the value of $R_1'$ and $R_2$ has the value of $R_2''$, which product with the formula ($I_B$) is isolated and, if desired, salified;
— or, when $R_2''$ represents a hydrogen atom, the product with the formula ($I_A$) is treated by a halogenide with the formula (V):

$$Hal{-}R_2' \qquad (V)$$

in which Hal represents a chlorine, bromine or iodine atom, and $R_2'$ has the significance of $R_2$ already

indicated with the exception of hydrogen, so as to obtain a product with the formula ($I_C$):

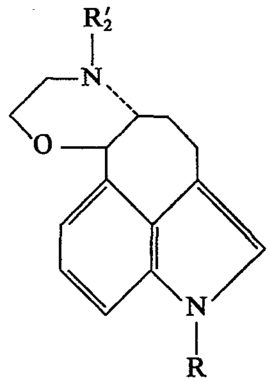

($I_C$)

in which R and $R_2'$ have the significance already indicated, corresponding to a product with the formula (I), in which $R_2$ has the values of $R_2'$ and $R_1$ represents a hydrogen atom, which product with the formula ($I_C$) either is isolated and, if desired, salified, or is treated by means of a halogenation agent, so as to obtain a product with the formula ($I_D$):

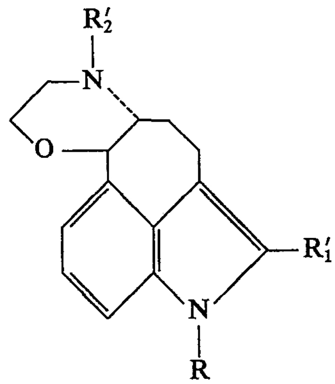

($I_D$)

in which R, $R_1'$ and $R_2'$ have the significance already indicated, corresponding to a product with the formula (I), in which $R_1$ has the values of $R_1'$ and $R_2$ has the values of $R_2'$, which product with the formula ($I_D$) is isolated and, if desired, salified;

— or, when $R_2''$ represents a hydrogen atom, the product with the formula ($I_A$) is treated by a formylation agent so as to obtain a product with the formula (VI):

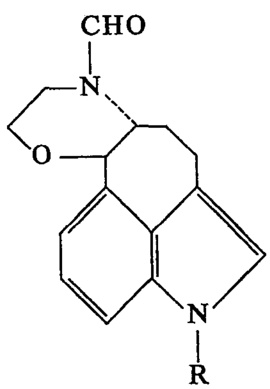

(VI)

in which R has the significance already indicated, which is treated by means of a reducing agent so as

to obtain a product with the formula ($I_E$)

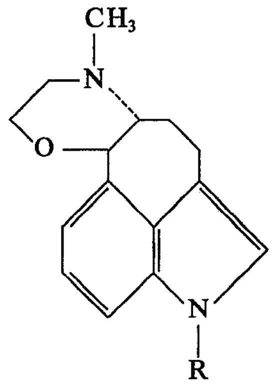

($I_E$)

in which R has the significance already indicated, corresponding to a product with the formula (I), in which $R_1$ represents a hydrogen atom and $R_2$ represents a methyl radical, which product with the formula ($I_E$)

either is isolated and, if desired, salified;

or is treated by means of a halogenation agent so as to obtain a product with the formula ($I_F$):

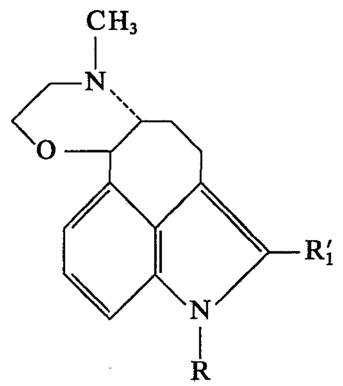

($I_F$)

in which R and $R_1'$ have the significance already indicated, corresponding to a product with the formula (I), in which $R_1$ has the values of $R_1'$ and $R_2$ represents a methyl radical, which product with the formula ($I_F$) is isolated and, if desired, salified,

— or, when R represents a hydrogen atom and $R_2''$ has the values as defined previously with the exception of hydrogen, the product with the formula ($I_A$) is treated by a halogenide with the formula

Hal—R′

in which Hal represents a chlorine, bromine or iodine atom and R′ has the significance of R, with the exception of hydrogen, so as to obtain a product with the formula ($I_G$):

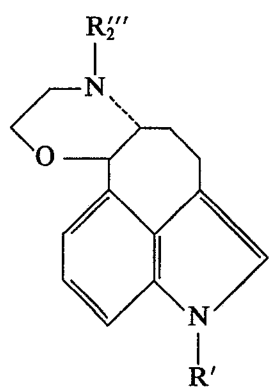

($I_G$)

in which R′ has the significance already indicated and $R_2'''$ has the significance of $R_2''$, with the exception of hydrogen, corresponding to a product with the formula (I), in which R has the values of R′ and $R_2$ has the values of $R_2'''$, which product with the formula ($I_G$) either is isolated and, if desired,

salified, or is treated by a halogenation agent, so as to obtain a product with the formula ($I_H$):

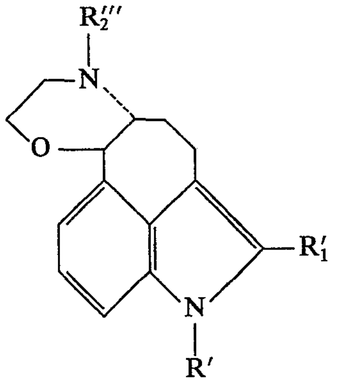

($I_H$)

in which R′, $R_1'$ and $R_2'''$ have the significance already indicated, corresponding to a product with the formula (I), in which R has the values of R′, $R_1$ has those of $R_1'$ and $R_2$ has those of $R_2'''$, which product with the formula ($I_H$) is isolated and, if desired, salified.

2. Process according to Claim 1, characterized in that
— cyclization of the product with the formula (III) is carried out by means of sodium hydride;
— the product with the formula (IV) is reduced by means of aluminium-lithium hydride;
— the halogenation agent is, in the case of bromation, the bromated complex of 2-pyrrolidone with the formula:

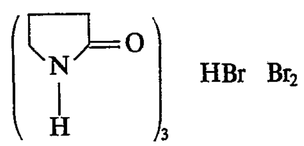

3. Process according to Claim 1 or 2, characterized in that a product with the formula (II) in which R represents a hydrogen atom, a methyl radical or a benzyl radical and $R_2''$ represents a hydrogen atom or an alkyl radical containing 1—5 carbon atoms or a cycloalkylalkyl radical containing 4—7 carbon atoms is used at the start.

4. Process according to Claim 1 or 2, characterized in that a product with the formula (II) in which $R_2''$ represents an alkyl radical containing 1—5 carbon atoms or a cycloalkylalkyl containing 4—7 carbon atoms and a product with the formula (VI) in which R′ represents a methyl radical or a benzyl radical are used at the start.

5. Process according to Claim 1 or 2, characterized in that a product with the formula (II) in which R represents a hydrogen atom and $R_2''$ represents a hydrogen atom or an alkyl radical containing 1—5 carbon atoms is used at the start.